# EUROPEAN PATENT APPLICATION

(11) **EP 4 570 904 A1**
(43) Date of publication of application: **18.06.2025**
(21) Application number: 23852538.0
(22) Date of filing: 07.08.2023
(51) Int. Cl.: C12N 5/09, C12N 1/00, C12Q 1/02

(54) **METHOD FOR CULTURING CANCER ORGANOID AND METHOD FOR SCREENING TEST SUBSTANCE**

(30) Priority: 08.08.2022 JP 2022126547
(71) Applicant: Toppan Holdings Inc., Tokyo 110-0016 (JP); Japanese Foundation For Cancer Research, Tokyo 135-8550 (JP); OSAKA UNIVERSITY, Suita-shi Osaka 565-0871 (JP)
(72) Inventor: MORIMURA, Rii, Tokyo 110-0016 (JP); SHINOZAKI, Eiji, Tokyo 135-8550 (JP); KITANO, Shiro, Tokyo 110-0016 (JP); YAO, Ryoji, Tokyo 135-8550 (JP); MATSUSAKI, Michiya, Suita-shi, Osaka 565-0871 (JP)
(74) Representative: TBK
(86) International application number: PCT/JP2023/028787
(87) International publication number: WO 2024/034576

(57) **Abstract**

This method for culturing a cancer organoid includes culturing the cancer organoid on a top surface or inside of a cell structure in a medium not including an extracellular matrix, and the cell structure includes cells constituting stroma, and has two or more cell layers laminated in a thickness direction. A method for screening a test substance includes culturing a cell structure including the cancer organoid obtained by the method for culturing a cancer organoid in a presence of the test substance, and evaluating an effect of the test substance on the cancer organoid.

## Description

### TECHNICAL FIELD

The present invention relates to a method for culturing a cancer organoid and method for screening a test substance.

Priority is claimed on Japanese Patent Application No. 2022-126547, filed August 8, 2022, the content of which is incorporated herein by reference.

### BACKGROUND ART

In the related art, in cancer research, experiments using established cancer cell lines that have been subcultured under optimized conditions for culture have been the mainstream. However, cancer cell lines that have been maintained and cultured for a long time in vitro may not necessarily reflect the properties of clinical tumors or the response to drugs. Therefore, for the development of an anti-cancer agent with higher accuracy and the selection of the optimal treatment for each patient, cancer organoids derived from the tumor tissue of the patient (also referred to as PDO) are considered promising (for example, refer to Non Patent Document 1).

The PDO retains many of the properties of the tumor tissue of the patient from which the PDO is derived even during the culture. On the other hand, in vivo, the microenvironment of cancer is composed of various cells such as cells constituting the stroma. In contrast, cancer organoids require an extracellular matrix (also referred to as ECM) such as Matrigel (registered trademark) (BD Biosciences) for culture. Since the cells constituting the stroma cannot remain in the extracellular matrix, cancer organoids cannot be co-cultured in contact with the cells constituting the stroma or the blood vessels composed of the cells. Therefore, the interaction between the cancer organoids and the cells constituting the stroma and the like cannot be evaluated (for example, refer to Non Patent Document 1).

In addition, a dedicated special medium is required for culturing cancer organoids, but growth factors and low-molecular-weight inhibitors contained in the medium may affect gene expression and signaling pathways of the cancer organoids, which may affect drug sensitivity.

### Citation List

### Non Patent Document

Non Patent Document 1: Veninga V et al., "Tumor organoids: Opportunities and challenges to guide precision medicine.", Cancer Cell, Vol. 39, Issue 9, pp. 1190-1201, 2021

### SUMMARY OF INVENTION

### Technical Problem

The present invention has been made in view of the above circumstances, and provides a method for culturing a cancer organoid capable of co-culturing a cancer organoid and cells constituting stroma without using an extracellular matrix, while maintaining the properties of the cancer organoid, and capable of evaluating cancer cell infiltration, and a method for screening a test substance using the cancer organoid obtained by the method for culturing a cancer organoid.

### Solution to problem

That is, the present invention includes the following aspects.
(1) A method for culturing a cancer organoid including: culturing the cancer organoid on a top surface or inside of a cell structure in a medium not including an extracellular matrix, in which the cell structure includes cells constituting stroma, and has two or more cell layers laminated in a thickness direction.
(2) The method for culturing a cancer organoid according to (1), in which the culturing includes dispersing the cancer organoid as a single cell and then culturing the cancer organoid on the top surface or inside of the cell structure.
(3) The method for culturing a cancer organoid according to (1) or (2), in which the culturing includes seeding the cancer organoid on a top surface of a first cell structure having two or more cell layers laminated in the thickness direction, then further seeding a cell suspension containing the cells constituting the stroma to construct a second cell structure on an upper part of the cancer organoid, and culturing the cancer organoid on the inside of the cell structure.
(4) The method for culturing a cancer organoid according to any one of (1) to (3), in which the medium is a medium consisting of any one or a combination of two or more of D-MEM, ESC, EBM2, EGM2 not containing Hidrocortisone as an added factor, RPMI-1640, Ham's F-12, and Mccoy's 5a.
(5) The method for culturing a cancer organoid according to any one of (1) to (4), in which the cancer organoid has a ductal structure.
(6) The method for culturing a cancer organoid according to any one of (1) to (5), in which the cell structure includes one or more types of cells selected from the group consisting of fibroblasts, pericytes, endothelial cells, and immune cells as the cells constituting the stroma.
(7) The method for culturing a cancer organoid according to (6), in which the endothelial cells are one or more types of cells selected from the group consisting of vascular endothelial cells and lymphatic endothelial cells.
(8) The method for culturing a cancer organoid according to any one of (1) to (7), in which the cell structure has a vascular network structure.
(9) The method for culturing a cancer organoid according to any one of (1) to (8), in which in the cell structure, ten or more cell layers are laminated in the thickness direction.
(10) A method for screening a test substance including: culturing a cell structure including the cancer organoid obtained by the method for culturing a cancer organoid according to any one of (1) to (9) in a presence of the test substance; and evaluating an effect of the test substance on the cancer organoid.

### Advantageous Effects of Invention

According to the method for culturing a cancer organoid according to the above-described aspect, a cancer organoid and cells constituting stroma can be co-cultured without using an extracellular matrix, while maintaining the properties of the cancer organoid, and cancer cell infiltration can be evaluated.

### BRIEF DESCRIPTION OF DRAWINGS

[FIG. 1A] A schematic configuration view showing a method for culturing a cancer organoid according to an embodiment of the present invention.
[FIG. 1B] A schematic configuration view showing a method for culturing a cancer organoid according to another embodiment of the present invention.
[FIG. 1C] A schematic configuration view showing a method for culturing a cancer organoid according to still another embodiment of the present invention.
[FIG. 1D] A schematic configuration view showing a method for culturing a cancer organoid according to still another embodiment of the present invention.
[FIG. 2A] Fluorescent immunostaining images using an anti-EpCAM antibody in a case where single-celled patient-derived cancer organoids (PDOs) are cultured on a top surface of a cell structure in Example 1.
[FIG. 2B] A graph showing the confluency (%) of PDOs in a case where single-celled PDOs are cultured on the top surface of the cell structure in Example 1.
[FIG. 3A] Fluorescent immunostaining images using an anti-EpCAM antibody in a case where single-celled PDOs are cultured inside the cell structure in Example 1.
[FIG. 3B] A graph showing the confluency (%) of PDOs in a case where single-celled PDOs are cultured inside the cell structure in Example 1.
[FIG. 3C] Immunostaining images of sections using an anti-EpCAM antibody and an anti-CD31 antibody in a case where single-celled PDOs are cultured inside the cell structure in Example 1.
[FIG. 3D] Fluorescent immunostaining images of sections using an anti-EpCAM antibody and an anti-CD31 antibody in a case where single-celled PDOs are cultured inside the cell structure in Example 1.
[FIG. 3E] Fluorescent immunostaining images of sections using an anti-CD31 antibody in a case where single-celled PDOs are cultured inside the cell structure in Example 1.
[FIG. 4A] Bright field images observed from the top surface in a case where PDOs maintaining the morphology are cultured on the top surface of the cell structure in Example 1.
[FIG. 4B] Immunostaining images of sections using an anti-EpCAM antibody in a case where PDOs maintaining the morphology are cultured on the top surface of the cell structure in Example 1.
[FIG. 5] Graphs showing the results of a drug efficacy evaluation test using AMG 510 as a single agent or a combination of AMG 510 and cetuximab under the condition in which single-celled PDOs are cultured on the top surface of the cell structure in Example 2.
[FIG. 6] Immunostaining images of tissues cultured in each medium in Example 3 using an anti-CD31 antibody.
[FIG. 7] Immunostaining images of the tissues cultured in each medium in Example 3 using an anti-CD31 antibody.
[FIG. 8] Immunostaining images of the tissues cultured in each medium in Example 3 using an anti-CD31 antibody.
[FIG. 9] Immunostaining images of the tissues cultured in each medium in Example 3 using an anti-CD31 antibody.
[FIG. 10] Immunostaining images of the tissues cultured in each medium in Example 3 using an anti-CD31 antibody.
[FIG. 11] Immunostaining images of the tissues cultured in each medium in Example 3 using an anti-CD31 antibody.
[FIG. 12] Immunostaining images of sections of tissues in which HCT26-1T and HCT26-3LM are cultured in each medium in Example 4 using an anti-EpCAM antibody.
[FIG. 13] Graphs showing a change over time in confluency (%) of PDOs in tissues in which HCT26-1T and HCT26-3LM are cultured in each medium in Example 4.
[FIG. 14] Immunostaining images of PDOs in sections of tissues in a case where HCT26-1T is cultured on the top surface of the cell structure in Example 4 using an anti-EpCAM antibody.
[FIG. 15] Immunostaining images of PDOs in sections of tissues in a case where HCT26-3LM is cultured on the top surface of the cell structure in Example 4 using an anti-EpCAM antibody.
[FIG. 16] Immunostaining images of PDOs in sections of tissues in a case where HCT26-1T is cultured inside the cell structure in Example 4 using an anti-EpCAM antibody.
[FIG. 17] Immunostaining images of PDOs in sections of tissues in a case where HCT26-3LM is cultured inside the cell structure in Example 4 using an anti-EpCAM antibody.

### DESCRIPTION OF EMBODIMENTS

### <<Method for Culturing Cancer Organoid>>

A method for culturing a cancer organoid according to an embodiment of the present invention (hereinafter, sometimes simply referred to as a "culturing method of the present embodiment") includes culturing the cancer organoid on a top surface or inside of a cell structure in a medium not including an extracellular matrix (hereinafter, sometimes referred to as a "culture step"), and the cell structure includes cells constituting stroma (hereinafter, referred to as "stromal cells"), and has two or more cell layers laminated in a thickness direction.

The cell structure used in the culturing method of the present embodiment is a structure that includes stromal cells and imitates a stromal tissue. The cancer organoid is cultured in a medium that does not include an extracellular matrix as a culture medium on the top surface or inside of a cell structure that imitates the stroma, instead of a normal culturing method in which the cancer organoid is attached to an extracellular matrix such as Matrigel and then cultured. Therefore, cancer organoids and stromal cells that cannot be co-cultured due to the presence of an extracellular matrix in the related art can be co-cultured.

In addition, in the culturing method of the present embodiment, on the top surface or inside of the cell structure that imitates the stromal tissue, the cancer organoid can be cultured without using an excessive growth factor or an artificial inhibitor, in addition to not containing the extracellular matrix as the culture medium. As a result, a cancer organoid-containing cell structure in which the cancer organoid is included on the top surface or inside of the cell structure can be obtained. That is, a culture obtained by the present embodiment (that is, the cancer organoid-containing cell structure) is a culture obtained under an environment closer to a living body. Therefore, as shown in Examples described later, in a case where the cancer organoid has a ductal structure in the culture and is a cancer organoid derived from a patient, the properties of the tumor tissue of the patient from which the cancer organoid is derived can be maintained. Therefore, the obtained culture is very useful as a sample for evaluating the drug efficacy of a candidate substance for an anti-cancer agent under conditions closer to the living body and for selecting an optimal anti-cancer agent for each patient.

Further, as shown in Examples described later, in the culturing method of the present embodiment, since the cancer organoid is observed to be infiltrated into the cell structure, the obtained culture can be used for evaluating cancer cell infiltration.

### <Cancer Organoid>

The cancer organoid used in the culturing method of the present embodiment may be prepared using a cultured cell line, but the cancer organoid is preferably prepared using a tumor tissue or cancer cell of a cancer patient or a cancer animal, and is more preferably an organoid derived from a cancer patient, since the organoid reflects the properties of a clinical tumor or the reactivity to a drug.

**In** the present embodiment and the present specification, the cancer cells refer to cells derived from somatic cells and acquired the ability to proliferate indefinitely, and is a malignant neoplasm that is infiltrated into surrounding tissues or causes metastasis.

The cancer organoid may consist of a plurality of types of cells or may consist of only a specific type of cell. In addition, the cancer organoid may consist of only cancer cells, or may consist of cancer cells and cells other than cancer cells.

The tumor tissue or cancer cell from which the cancer organoid is derived may be a tissue or cell collected from an animal of any biological species. For example, tumor tissues or cancer cells collected from humans and animals such as monkeys, marmosets, dogs, cats, rabbits, pigs, cows, horses, sheep, goats, mice, and rats can be used.

The tumor tissue from which the cancer organoid is derived may be a solid tissue or a liquid tissue, but a solid tissue is preferable. Examples of the solid tissue include those obtained by surgically excising and collecting epithelial tissues, connective tissues, muscle tissues, nervous tissues, stromal tissues, and mucosal tissues. Examples of the liquid tissue include body fluids such as blood, lymph, pleural effusions, ascitic fluids, cerebrospinal fluids, tears, saliva, and urine. For example, these tissues can be removed with a scalpel, laser or the like in surgery, endoscopy or the like, or collected with a syringe, a swab or the like. As the human tumor tissue, for example, a tissue collected for clinical examination can be used.

The cancer from which the main tissue or the cancer cell is derived is preferably a solid cancer. Examples of the solid cancer include, but not limited to, breast cancer (for example, invasive ductal carcinoma, ductal carcinoma in situ, and inflammatory breast cancer), prostate cancer (for example, hormone-dependent prostate cancer and hormone-independent prostate cancer), pancreatic cancer (for example, pancreatic duct cancer), gastric cancer (for example, papillary adenocarcinoma, mucinous adenocarcinoma, and adenosquamous carcinoma), lung cancer (for example, non-small-cell lung cancer, small-cell lung cancer, and malignant mesothelioma), colon cancer (for example, gastrointestinal stromal tumor), rectal cancer (for example, gastrointestinal stromal tumor), colorectal cancer (for example, familial colorectal cancer, hereditary nonpolyposis colorectal cancer, and gastrointestinal stromal tumor), small intestinal cancer (for example, non-Hodgkin's lymphoma and gastrointestinal stromal tumor), esophageal cancer, duodenal cancer, tongue cancer, pharyngeal cancer (for example, nasopharyngeal cancer, oropharyngeal cancer, and hypopharyngeal cancer), head and neck cancer, salivary gland cancer, brain tumor (for example, pineal astrocytoma, pilocytic astrocytoma, diffuse astrocytoma, and anaplastic astrocytoma), neurilemmoma, liver cancer (for example, primary liver cancer and extrahepatic bile duct cancer), renal cancer (for example, renal cell cancer and transitional cell cancer of the renal pelvis and ureter), gallbladder cancer, bile duct cancer, pancreatic cancer, hepatoma, endometrial cancer, cervical cancer, ovarian cancer (for example, epithelial ovarian cancer, extragonadal germ cell tumor, ovarian germ cell tumor, and ovarian low-malignant potential tumor), bladder cancer, urethral cancer, skin cancer (for example, intraocular (ocular) melanoma and Merkel cell carcinoma), melanoma (malignant melanoma), thyroid cancer (for example, medullary thyroid cancer), parathyroid cancer, nasal cancer, paranasal cancer, retinal sarcoma, penile cancer, testicular tumor, pediatric solid cancer (for example, Wilms tumor and pediatric renal tumor), Kaposi sarcoma, Kaposi sarcoma caused by AIDS, tumor of the maxillary sinus, leiomyosarcoma, and rhabdomyosarcoma.

For example, cancer patient-derived organoids can be prepared by the following method. That is, first, a tumor tissue of a cancer patient is surgically excising, immediately cut into small pieces, and washed with PBS. Next, the small pieces of the tumor tissue are dissociated at a temperature of about 37°C by adding an enzyme such as a digestion buffer collagenase and dispase. Next, the tumor tissue is vigorously stirred by pipetting in PBS, and the tumor tissue pieces are recovered and centrifuged. The PDO is obtained by suspending the pellet after centrifugal separation in an extracellular matrix such as Matrigel, transferring the suspension to a culture vessel, and culturing the suspension.

Alternatively, cancer organoids may be prepared using cancer cells isolated from a tumor tissue of a cancer patient. As a method of isolating cancer cells from a tumor tissue, for example, as a method of isolating cancer cells from an epithelial tissue, methods known in the art may be used. For example, a crypt can be isolated by leaving a chelating agent and a tumor tissue at a constant temperature. After washing the tissue, the epithelial cell layer is peeled off from the submucosal layer with a glass slide and finely chopped. Thereafter, the cells are left to stand in a liquid containing trypsin or preferably at least one of EDTA and EGTA at a constant temperature. Thereafter, for example, the undigested tissue fragments and the single cells derived from the crypt are separated from each other by using at least one of filtration or a centrifugal machine. Instead of trypsin, another proteolytic enzymes, for example, at least one of collagenase and dispase I may be used. The same method is used to isolate fragments of the pancreas and the stomach.

In a case where only cancer cells are sorted from a tumor tissue derived from a cancer patient to prepare cancer organoids, before the cancer cells are sorted, the amount of cancer cells contained in the tumor tissue may be confirmed. The cancer cells can be sorted using the expression of a cancer cell-specific protein or an increase in enzyme activity as a cancer marker. The cancer marker is not particularly limited, and may be, for example, a protein that is specifically expressed in cancer cells, such as EpCAM, CEA, Cytokeratin, and HER2. In a case where these proteins are used as cancer markers, cancer cells can be visualized by immunohistochemistry (also referred to as IHC) staining or immunofluorescence (also referred to as IF) staining using antibodies against these proteins. In addition, in a case where the enzyme activity of γ-glutamyl transpeptidase or β-galactosidase, which is elevated in cancer cells, is used as a cancer marker, these enzyme activities can be measured using a fluorescent probe such as ProteoGREEN (registered trademark, manufactured by GORYO Chemical, Inc.) and GlycoGREEN (registered trademark, manufactured by GORYO Chemical, Inc.).

The sorting of cancer cells can be performed by a method such as flow cytometry, magnetic separation, dielectrophoresis, size fractionation, or density gradient fractionation. The sorting method can be appropriately determined based on the organ from which the original patient tumor is derived, the clinical background, various previous test results, and the like. In a case of using flow cytometry, cancer cells can be sorted by separating cells that are positive in a staining treatment, after staining with IF or a fluorescent probe. In addition, in flow cytometry, since live cells can also be distinguished from dead cells from the values of forward scattered light and side scattered light, it is possible to sort and recover live cancer cells more efficiently. Moreover, in a case of using magnetic separation, cells are magnetically labeled with an antibody, but any of a positive selection method that magnetically recovers labeled cancer cells and a negative selection method that magnetically removes labeled stromal cells can be selected. In addition, in a case of using dielectrophoresis and/or density gradient fractionation, it is preferable to understand in advance the dielectric characteristics and/or density gradient characteristics of the cell type used for the construction of stromal cells.

Examples of the method for preparing cancer organoids include the method described in "Sakahara M et al., "IFN/STAT Signaling Controls Tumorigenesis and the Drug Response in Colorectal Cancer.", Cancer Sci., , Vol. 110, Issue 4, pp. 293-1305, 2019. (Reference Document 1)".

The method is a method of culturing cancer cells or a tumor tissue containing the cancer cells by attaching or embedding the cancer cells or the tumor tissue containing the cancer cells to an extracellular matrix.

### [Extracellular Matrix (ECM)]

In general, the "extracellular matrix" means a supramolecular structure existing outside a cell in a living organism. This ECM serves as a scaffold for proliferation of cancer cells or a tumor tissue containing the cancer cells. The ECM contains various polysaccharides, water, elastin, and glycoproteins. Examples of the glycoproteins include collagen, entactin (nidogen), fibronectin, and laminin.

The ECM may be prepared using connective tissue cells or may be a commercially available ECM.

As a method for preparing ECM, for example, ECM can be used as a scaffold by culturing ECM-producing cells (for example, fibroblasts and the like), then taking out these cells, and adding cancer cells or a tumor tissue containing the cancer cells.

Examples of the ECM-producing cells include chondrocytes that mainly produce collagen and proteoglycan, fibroblasts that mainly produce type IV collagen, laminin, interstitial procollagen, and fibronectin, and colonic myofibroblasts that mainly produce collagen (type I, type III, and type V), chondroitin sulfate proteoglycan, hyaluronic acid, fibronectin, and tenascin-C.

Examples of the commercially available ECM that is generally used for culturing cancer organoids include synthetic ECMs such as an extracellular matrix protein (manufactured by Invitrogen), a basement membrane preparation (for example, Matrigel (registered trademark) (BD Biosciences)) derived from Engelbreth-Holm-Swarm (also known as EHS) mouse sarcoma cells, and ProNectin (Sigma Z378666). In addition, a mixture of natural ECM and synthetic ECM may be used.

After gelation (or coagulation) of the ECM, cancer cells or a tumor tissue containing the cancer cells may be placed thereon. Alternatively, the ECM and the cancer cells or the tumor tissue containing the cancer cells may be mixed, and then the mixture may be seeded in a culture vessel to be gelated (or coagulated). Alternatively, the ECM may be used by adding a medium when the ECM is gelated (or coagulated) at 37°C, and diffusing the cancer cells or the tumor tissue containing the cancer cells in the ECM. The cancer cells or the tumor tissue containing the cancer cells in the medium can be attached to the ECM by interacting with the surface structure (for example, integrin) of the ECM.

The ECM may be used by coating the culture vessel or the like with the ECM.

The cancer organoids can be formed by culturing the cancer cells or the tumor tissue containing the cancer cells in a state of being attached to ECM. The culture temperature is preferably 30°C or higher and 40°C or lower, and more preferably about 37°C. The culture time can be appropriately adjusted depending on the cells used. In addition, the CO₂ concentration in the culture atmosphere is preferably about 0.03% by volume or more and 10% by volume or less, and most preferably about 5% by volume, with respect to 100% by volume of the culture atmosphere. In addition, the cells can also be cultured in an environment in which the O₂ concentration is controlled to be lower than that of the atmospheric air. In addition, examples of the medium used for culturing the cancer organoids include a medium generally used for culturing organoids (for example, IntestiCult Organoid Growth Medium (Human) manufactured by STEMCELL Technologies.).

### <Cell Structure>

**In** the present embodiment and the present specification, the "cell structure" means a three-dimensional cell aggregate including at least stromal cells. The "thickness of the cell structure" means the length of the structure in a self-weight direction. The self-weight direction isthe direction of gravity and is also referred to as a thickness direction. More specifically, the thickness of the cell structure is the thickness of a section taken along a line passing through the center of gravity when viewed from the upper surface of the cell structure, and is the thickness of the section in the substantially central portion of the cell structure. The shape of the cell structure varies depending on a vessel used for producing the cell structure, but for example, in a case where the cell structure is produced using a cell culture insert having a cylindrical shape, the cell structure has a cylindrical shape. In this case, the shape of the cell structure when viewed from the upper surface is a circle, and the center of gravity when viewed from the upper surface is the center of the circle. The shape of the cell structure is not limited to the cylindrical shape and can be any shape depending on the purpose. Specifically, for example, a polygonal prism shape such as a triangular prism shape and a quadrangular prism shape can be adopted. The "cell layer" means a layer composed of a group of cells and stroma which are present in a direction perpendicular to the thickness direction with the cell nuclei not overlapping each other in the thickness direction.

The cell structure may include a first cell structure and a second cell structure as described later. In such a cell structure, the cancer organoid is sandwiched between the first and second cell structures. In a case where the cell structure includes the first cell structure and the second cell structure, the thickness of the cell structure is the sum of the thickness of the first cell structure and the thickness of the second cell structure.

The cells such as the stromal cells constituting the cell structure are not particularly limited, and may be cells collected from an animal, cells cultured from cells collected from an animal, cells obtained by subjecting cells collected from an animal to various treatments, or cultured cell lines. In addition, commercially available cells may be used as cells such as stromal cells constituting the cell structure, or patient-derived cells may be used. In a case where cells are collected from an animal, a collection site is not particularly limited, and the cells may be somatic cells derived from bone, muscle, an internal organ, a nerve, the brain, skin, blood, or the like, may be a germ cell, or may be embryonic stem cells (also referred to as ES cells).

In addition, the biological species from which the cells constituting the cell structure are derived is not particularly limited, and for example, cells derived from humans and animals such as monkeys, dogs, cats, rabbits, pigs, cows, mice, and rats can be used. The cells obtained by culturing cells obtained from animals may be primary cultured cells or subcultured cells. In addition, the cells obtained by applying various treatments may include induced pluripotent stem cells (also referred to as iPS cells) or cells after differentiation induction.

The cell structure may be composed of only cells derived from the same organism species, or cells derived from a plurality of types of organism species.

Examples of stromal cells that construct the cell structure include endothelial cells, fibroblasts, pericytes, immune cells, nerve cells, mast cells, epithelial cells, cardiac muscle cells, liver cells, islet cells, tissue stem cells and smooth muscle cells. Immune cells are cells involved in immunity. Specific examples thereof include lymphocytes, macrophages and dendritic cells. Examples of lymphocytes include T cells, B cells, NK cells and plasma cells. The stromal cells contained in the cell structure may be one or more types. The stromal cells contained in the cell structure preferably include one or more selected from the group consisting of fibroblasts, pericytes, endothelial cells, and immune cells.

The number of stromal cells in the cell structure is not particularly limited, but since a cell structure that more closely imitates the stromal tissue can be formed, the abundance ratio (cell number ratio) of stromal cells with respect to all the cells constituting the cell structure is preferably 30% or more, more preferably 50% or more, still more preferably 70% or more, and even more preferably 80% or more.

A blood vessel network structure and a lymphatic network structure are considered to be important for the cell structure to exhibit functions similar to those of the stromal tissue in the living body. Therefore, the cell structure is preferably a cell structure having a vascular network structure. That is, the cell structure is preferably a cell structure in which a vascular network structure such as of lymphatic vessels and/or blood vessels is three-dimensionally constructed inside a laminate of non-vascularized cells to construct tissues closer to those in the living body. The vascular network structure may be formed only inside the cell structure, or may be formed so that at least part of the vascular network structure is exposed on the front surface or the bottom surface of the cell structure. In addition, the vascular network structure may be constructed in the entire cell structure, or may be formed only in a specific cell layer.

In the present embodiment and the present specification, the "vascular network structure" refers to a network structure, such as a blood vessel network or a lymphatic vessel network, in living tissues.

The vascular network structure can be formed by incorporating endothelial cells constituting vessels as stromal cells. The endothelial cells contained in the cell structure may be vascular endothelial cells or lymphatic endothelial cells. In addition, the endothelial cells contained in the cell structure may include both vascular endothelial cells and lymphatic endothelial cells.

In a case where the cell structure includes a vascular network structure, it is preferable that the cells other than the endothelial cells in the cell structure are cells that constitute surrounding tissues of vessels in the living body, since the endothelial cells can easily form a vascular network maintaining the original function and shape. Since it is possible to more closely approximate the stromal tissue in the living body and the environment in the vicinity of the stromal tissue in the living body, the cells other than the endothelial cells are more preferably cells including at least fibroblasts, and still more preferably cells including vascular endothelial cells and fibroblasts, cells including lymphatic endothelial cells and fibroblasts, or cells including vascular endothelial cells, lymphatic endothelial cells, and fibroblasts. The cells other than the endothelial cells contained in the cell structure may be cells derived from the same biological species as the endothelial cells or may be cells derived from a different biological species.

The number of endothelial cells in the cell structure is not particularly limited as long as the number is a sufficient number for forming the vascular network structure, and can be appropriately determined in consideration of the size of the cell structure, the cell type of the endothelial cells and the cells other than the endothelial cells. For example, by setting the abundance ratio (cell number ratio) of the endothelial cells to the total cells constituting the cell structure to 0.1% or more, a cell structure in which a vascular network structure is formed can be prepared. In a case where fibroblasts are used as the cells other than the endothelial cells, the number of endothelial cells in the cell structure is preferably 0.1% or more and more preferably 0.1% or more and 5.0% or less of the number of fibroblasts. In a case where both the vascular endothelial cell and the lymphatic endothelial cell are included as the endothelial cells, the total number of the vascular endothelial cells and the lymphatic endothelial cells is preferably 0.1% or more and more preferably 0.1% or more and 5.0% or less of the number of the fibroblasts.

In a case where the culture obtained by the culturing method of the present embodiment is used in a cell-based assay such as a drug susceptibility test, it is preferable that the cell structure to be used is a structure closer to the stroma in the living body. Therefore, the cell structure is preferably one in which a vascular network structure is formed, more preferably a cell structure in which a vascular network structure is formed and fibroblasts are included, and still more preferably a cell structure in which a vascular network structure is formed, fibroblasts are included, and ten or more cell layers are laminated in the thickness direction.

In addition, in order to meet the demand for observing the infiltration of cancer cells into the blood vessels, it is preferable that the cell structure has a vascular network structure.

The size and shape of the cell structure are not particularly limited. Since a cell structure in a state closer to that of the stromal tissue in the living body can be formed and cancer organoid culture in an environment closer to the living body can be expected, the thickness of the cell structure is preferably 5 µm or more, more preferably 30 µm or more, still more preferably 100 µm or more, and even more preferably 150 µm or more. In addition, the thickness of the cell structure is preferably 500 µm or less, more preferably 400 µm or less, and still more preferably 200 µm or less.

The number of cell layers in the cell structure is preferably about 2 or more and 60 or less, more preferably about 3 or more and 60 or less, still more preferably about 5 or more and 60 or less, even more preferably about 5 or more and 20 or less, and particularly preferably about 10 or more and 20 or less.

The number of cell layers constituting the cell structure is measured by dividing the total number of cells that constitute a three-dimensional structure by the number of cells per layer (the number of cells necessary to form one layer). The number of cells per layer can be investigated in such a manner that cells are cultured in advance on a plane so that the cells are confluent in a cell culture vessel that is used in the production of a cell structure. Specifically, the number of cell layers in a cell structure formed in a certain cell culture vessel can be calculated by measuring the total number of cells that constitute the cell structure, and dividing the total number of cells by the number of cells per layer in the cell culture vessel.

In general, the cell structure is constructed in a cell culture vessel. The cell culture vessel is not particularly limited as long as the vessel enables the construction of a cell structure, and culture of the constructed cell structure. Specifically, examples of the cell culture vessel include dishes, cell culture inserts (for example, a Transwell (registered trademark) insert, a Netwell (registered trademark) insert, a Falcon (registered trademark) cell culture insert, a Millicell (registered trademark) cell culture insert, and the like), tubes, flasks, bottles, and plates. In the construction of the cell structure, dishes or various cell culture inserts are preferable, since the constructed cell structure can be used as it is for culturing the cancer organoid.

The cell structure may be a structure formed from multiple cell layers of two or more layers containing stromal cells, and the method for constructing the cell structure is not limited. For example, the method may be a method for constructing a layer at a time and laminating the layers in sequence, or may be a method for constructing two or more cell layers at a time, or may be a method for constructing multiple cell layers by appropriately combining both of the former methods.

In addition, the cell structure may be a multilayered structure in which the cell types constituting each cell layer are different for each layer, or the cell types constituting each cell layer may be cell types common to all the layers of the structure. For example, a method in which a layer is formed for each cell type, and the cell layers are laminated in sequence, or a method in which a cell mixture solution obtained by mixing a plurality of types of cells is prepared in advance, and a cell structure having a multilayered structure is constructed at a time from the cell mixture solution may be used.

Examples of the construction method by constructing one layer at a time and then laminating the layers in sequence include the method described in Japanese Patent No. 4919464 (Reference Document 2), that is, a method for laminating cell layers continuously by alternately repeating a step of forming a cell layer, and a step of bringing the formed cell layer into contact with a solution containing an extracellular matrix (ECM) component. For example, when performing this method, a cell structure in which a vascular network structure is formed throughout the entire structure can be constructed by preparing a cell mixture in which all cells constituting the cell structure are mixed in advance, and forming each cell layer with this cell mixture. Further, by forming each cell layer for each cell type, a cell structure in which a vascular network structure is formed only in a layer formed from endothelial cells can be constructed.

Examples of the method for constructing two or more cell layers at a time include the method described in Japanese Patent No. 5850419 (Reference Document 3). This method is a method for constructing a cell structure formed from multiple cell layers by coating the entire cell surface in advance with a polymer containing an arginine-glycine-aspartic acid (RGD) sequence to which integrin binds and a polymer interacting with the polymer containing the RGD sequence, storing the coated cells coated with the adhesive film in a cell culture vessel and then accumulating the coated cells by centrifugation or the like. For example, in a case of performing the method, coated cells prepared by preparing a cell mixture in which all the cells constituting the cell structure are mixed in advance and adding an adhesive component to this cell mixture are used. Thus, a cell structure in which the cell composition is uniform throughout the entire structure by a single centrifugation treatment can be constructed.

The cell structure can also be constructed, for example, by the methods described in Japanese Patent No. 6639634 (Reference Document 4) and Patent International Publication No. WO2019/039457A (Reference Document 5), that is, methods having steps of the following steps (a) to (c):
(a) a step of obtaining a mixture by mixing cells and an extracellular matrix component in a cationic buffer solution;
(b) a step of seeding the mixture obtained in step (a) above in a cell culture vessel; and
(c) a step of obtaining a cell structure in which cells are laminated in multiple layers in the cell culture vessel after step (b) above.

In step (a), cells are mixed with a buffer solution containing a cationic substance (also referred to as a cationic buffer solution) and an extracellular matrix component to form cell aggregates from the cell mixture to thereby obtain a three-dimensional cell tissue with few large voids therein. In addition, since the obtained three-dimensional cell tissues are relatively stable, the cell tissues can be cultured for at least several days, and are unlikely to be damaged during medium replacement. Further, step (b) can also include precipitating, in a cell culture vessel, the cell mixture seeded in the cell culture vessel. The cell mixture may be actively precipitated by centrifugal separation or the like, or may be spontaneously precipitated.

In step (a), it is preferable to further mix the cells with a strong electrolyte polymer. When the cells are mixed with the cationic substance, the strong electrolyte polymer, and the extracellular matrix component, a thick three-dimensional cell tissue with few voids can be obtained even in a case where the cells are spontaneously precipitated, without requiring treatment, such as centrifugal separation, for actively gathering the cells in step (b).

Examples of the cationic buffer solution include a tris-hydrochloric acid buffer solution, a tris-maleic acid buffer solution, a bis-tris buffer solution, and HEPES. The concentration and pH of the cationic substance in the cationic buffer solution (for example, tris in a tris-hydrochloric acid buffer solution) are not particularly limited as long as they do not adversely affect the growth of the cells and construction of the cell structure. For example, the concentration of the cationic substance in the cationic buffer solution can be 10 mM or more and 100 mM or less, and is preferably 40 mM or more and 70 mM or less, and more preferably 50 mM. In addition, the pH of the cationic buffer solution can be 6.0 or more and 8.0 or less, and is preferably 6.8 or more and 7.8 or less and more preferably 7.2 or more and 7.6 or less.

Examples of the strong electrolyte polymer include, but are not limited to, glycosaminoglycans such as heparin, chondroitin sulfate (for example, chondroitin 4-sulfate, or chondroitin 6-sulfate), heparan sulfate, dermatan sulfate, keratan sulfate, hyaluronic acid, and the like; dextran sulfate, rhamnan sulfate, fucoidan, carrageenan, polystyrene sulfonic acid, polyacrylamide-2-methylpropanesulfonic acid, polyacrylic acid, and derivatives thereof. The mixture prepared in step (a) may be mixed with only one strong electrolyte polymer, or two or more strong electrolyte polymers in combination. In the construction of the cell structure, the strong electrolyte polymer is preferably a glycosaminoglycan. Further, at least one from among heparin, dextran sulfate, chondroitin sulfate, and dermatan sulfate may more preferably be used. In addition, the strong electrolyte polymer is more preferably heparin.

The amount of the strong electrolyte polymer mixed in the cationic buffer solution is not particularly limited as long as it does not adversely affect the growth of the cells and the construction of the cell structure. For example, the concentration of the strong electrolyte polymer in the cationic buffer solution can be more than 0 mg/mL (higher than 0 mg/mL) and less than 1.0 mg/mL, and is preferably 0.025 mg/mL or more and 0.1 mg/mL or less, and more preferably 0.05 mg/mL or more and 0.1 mg/mL or less. Moreover, a cell structure can be constructed by adjusting the above mixture without mixing the above strong electrolyte polymer.

Examples of the extracellular matrix component include collagen, laminin, fibronectin, vitronectin, elastin, tenascin, entactin, fibrillin, proteoglycan, and modifications or variants thereof. Examples of the proteoglycan include chondroitin sulfate proteoglycan, heparan sulfate proteoglycan, keratan sulfate proteoglycan, and dermatan sulfate proteoglycan. The mixture prepared in step (a) may be mixed with only one extracellular matrix component, or two or more extracellular matrix components in combination. In the construction of the cell structure, it is preferable to use collagen, laminin, or fibronectin, and it is more preferable to use collagen. Modified forms and variants of the extracellular matrix components mentioned above may also be used, as long as they do not adversely affect the cell growth and the formation of the cell structure.

The amount of extracellular matrix component mixed in the cationic buffer solution is not particularly limited, as long as it does not adversely affect the cell growth and the construction of the cell structure. For example, the concentration of the extracellular matrix component in the cationic buffer solution can be more than 0 mg/mL (higher than 0 mg/mL) and lower than 1.0 mg/mL, and is preferably 0.025 mg/mL or more and 0.1 mg/mL or less, and more preferably 0.05 mg/mL or more and 0.1 mg/mL or less.

The mixing ratio of the strong electrolyte polymer to the extracellular matrix component to be mixed in the cationic buffer solution is 1:2 to 2:1. In the construction of the cell structure, the mixing ratio of the strong electrolyte polymer to the extracellular matrix component is preferably 1:1.5 to 1.5:1, and more preferably 1:1.

A cell structure with sufficient thickness can be constructed by repeating steps (a) to (c). Specifically, the following process is repeated: as step (b), the mixture prepared in step (a) is seeded on the cell structure obtained in step (c), and step (c) is then performed. The cell composition of the mixture newly seeded on the cell structure obtained in step (c) may be the same as or different from the cell composition that constitute the already constructed cell structure.

In a case where steps (a) to (c) are repeated, the obtained cell structure may be cultured after step (c) and before step (b). The culture conditions, such as composition of the culture medium used in the culture, culture temperature, culture time, atmospheric composition during culture, and the like, are determined to be suitable for culture of the cells forming the cell structure. Examples of the culture medium include Dulbecco's modified Eagle's minimal essential medium (D-MEM), Eagle's minimal essential medium (E-MEM), minimal essential medium α (MEM α), RPMI-1640, and Ham's F-12.

After step (a), the following steps (a'-1) and (a'-2) may be performed, and then step (b) may be performed: (a'-1) a step of removing a liquid part from the obtained mixture, and obtaining cell aggregates, and (a'-2) a step of suspending the cell aggregates in a solution. Desired tissues can be obtained by performing steps (a) to (c) described above, but more homogenous tissues can be obtained by performing steps (a), (a'-1), (a'-2) and (b) in this order.

In addition, after step (a), the following steps (b'-1) and (b'-2) may be performed in place of step (b) above. More homogenous tissues can also be obtained by performing steps (b'-1) and (b'-2). Also in step (b'-2), precipitating the cell mixture seeded in the cell culture vessel in the cell culture vessel is included as in step (b). The cell mixture may be actively precipitated by centrifugal separation or the like, or may be spontaneously precipitated. In the present embodiment and the present specification, the "cell viscous body" refers to a gel-like cell aggregate as described in "Nishiguchi et al., Macromol Bioscience, 2015, vol. 15 (3), p. 312-317. (Reference Document 6)".

Step (b'-1) is a step of seeding the mixture obtained in step (a) in a cell culture vessel, and then removing a liquid component from the mixture to obtain a cell viscous body.

Step (b'-2) is a step of suspending the cell viscous body in a solvent in the cell culture vessel.

The solvent for preparing a cell suspension is not particularly limited unless it is toxic to the cells or unless it impairs the growth and functions of the cells, and water, a buffer solution, a cell culture medium, or the like can be used. Examples of the buffer solution include phosphate buffered saline (PBS), HEPES, and Hanks buffer solution. Examples of the culture medium include D-MEM, E-MEM, MEM α, RPMI-1640, and Ham's F-12. In a case where a cell culture medium is used as a solvent for preparing the cell suspension, the cells can be cultured without removing the liquid component in step (c) described below.

Step (c') which is a step of forming a cell layer on a substrate may be performed in place of step (c) above.

In step (c) and step (c'), the liquid component may be removed from the seeded mixture.

The method of the removal treatment of the liquid component in steps (c) and (c') is not particularly limited, as long as it does not adversely affect the cell growth and the construction of the cell structure. The method of removing a liquid component from a suspension of the liquid component and a solid component can be suitably performed by a method known to a person skilled in the art. Examples of the method include suction, centrifugal separation, magnetic separation, and filtration. For example, in a case where a cell culture insert is used as the cell culture vessel, the cell culture insert in which the mixture is seeded can be subjected to centrifugal separation treatment at 10°C at 400 × g for 1 minute to precipitate the cell mixture, and then the liquid component can be removed by suction.

Next, the steps in the culturing method of the present embodiment will be described in detail below.

### <Culture Step>

In the culture step, the cancer organoids are cultured on the top surface or inside of the cell structure in a medium that does not include an extracellular matrix.

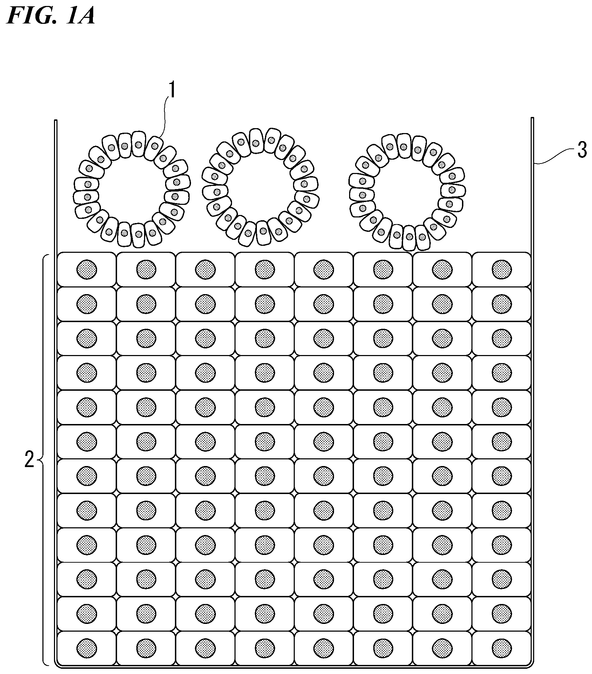

FIG. 1A is a schematic configuration view showing a method for culturing a cancer organoid according to an embodiment of the present invention (in a case where a cancer organoid is cultured on a top surface of a cell structure).

That is, in a case where cancer organoids 1 are cultured on a top surface of a cell structure 2, the cancer organoids 1 are added into a culture solution (the culture medium is not shown) of the cell structure constructed in a culture vessel 3. As a result, the cancer organoids 1 are attached to the top surface of the cell structure 2, and the cancer organoids 1 are cultured in a state of being attached to the top surface of the cell structure 2.

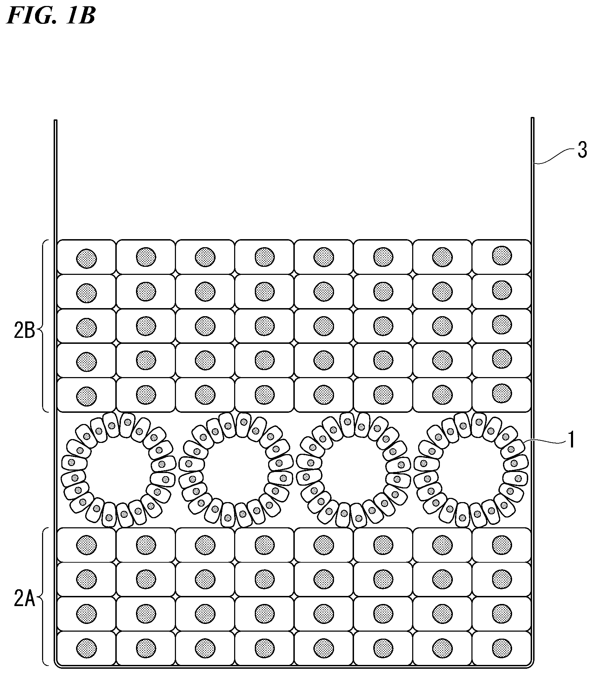

Alternatively, FIG. 1B is a schematic configuration view showing a method for culturing a cancer organoid according to another embodiment of the present invention (in a case where a cancer organoid is cultured inside a cell structure).

That is, in a case where the cancer organoids 1 are cultured inside the cell structure, the cancer organoids 1 are seeded on a top surface of a first cell structure 2A in which two or more cell layers are laminated in a thickness direction, then a cell suspension containing stromal cells is further seeded to construct a second cell structure 2B on the upper part of the cancer organoids 1, and the cancer organoids 1 are cultured inside the cell structure. The method described in the section of "Cell Structure" can be applied to the method for preparing the first cell structure 2A and the second cell structure 2B.

In the method described in FIGS. 1A and 1B, the culture can be performed while maintaining the morphology of the cancer organoid.

On the other hand, the cancer organoids can also be cultured on the top surface or inside of the cell structure after being dispersed as single cells. The number of cells to be seeded in the cell structure can be grasped by dispersing the cancer organoids as single cells. Therefore, in an assay for the drug efficacy evaluation or the like of a candidate substance for an anti-cancer agent using the obtained culture, the rate of remaining cells and the like can be calculated, and quantitative evaluation can be performed.

Hereinafter, the dispersion of the cancer organoids as single cells may be referred to as "single cell preparation", and the cancer organoids dispersed as single cells are sometimes referred to as "single-celled cancer organoids".

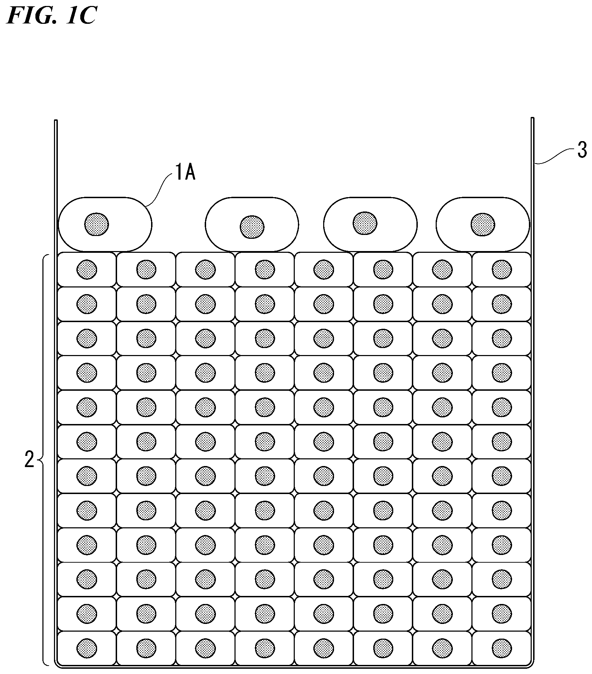

FIG. 1C is a schematic configuration view showing a method for culturing a cancer organoid according to another embodiment of the present invention (in a case where a single-celled cancer organoid is cultured on a top surface of a cell structure).

The culturing method is the same as the culturing method described in FIG. 1A, except that single-celled cancer organoids 1A are used in FIG. 1C.

That is, in a case where the single-celled cancer organoids 1A are cultured on the top surface of the cell structure 2, the single-celled cancer organoids 1A are added into the culture solution (the culture medium is not shown) of the cell structure constructed in the culture vessel 3. As a result, the single-celled cancer organoids 1A are attached to the top surface of the cell structure 2, and the single-celled cancer organoids 1A are cultured in a state of being attached to the top surface of the cell structure 2.

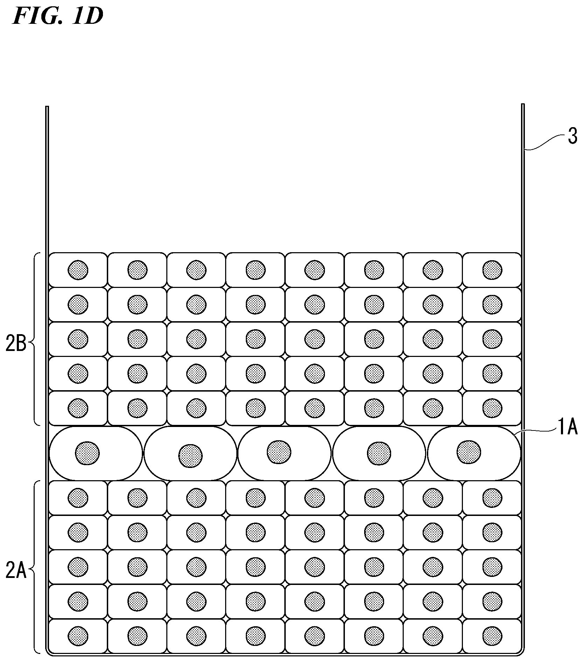

Alternatively, FIG. 1D is a schematic configuration view showing a method for culturing a cancer organoid according to still another embodiment of the present invention (in a case where a single-celled cancer organoid is cultured inside a cell structure).

That is, in a case where the single-celled cancer organoids 1A are cultured inside the cell structure, the single-celled cancer organoids 1A are seeded on the top surface of the first cell structure 2A in which two or more cell layers are laminated in the thickness direction, then a cell suspension containing stromal cells is further seeded to construct the second cell structure 2B on the upper part of the single-celled cancer organoids 1A, and the single-celled cancer organoids 1A are cultured inside the cell structure. The method described in the section of "Cell Structure" can be applied to the method for preparing the first cell structure 2A and the second cell structure 2B.

In the methods described in FIGS. 1C and 1D, examples of the method for obtaining a single-celled cancer organoid include an enzyme treatment method and the like. The enzyme used for the enzyme treatment may be one type or two or more types.

The enzyme to be used is not particularly limited as long as it is an enzyme having a target enzyme activity, and the enzyme may be an enzyme derived from any biological species or may be an artificial enzyme obtained by modifying a naturally occurring enzyme. In addition, the enzyme may be an enzyme extracted and purified from various cells or may be a chemically synthesized enzyme.

Specifically, as the enzyme used for the single cell preparation, one or more types of enzymes selected from the group consisting of trypsin, collagenase, dispase, elastase, papain, hyaluronidase, and alternative enzymes thereof are preferable, trypsin or an alternative enzyme thereof is more preferable, and an alternative enzyme of trypsin is still more preferable. Examples of the alternative enzyme of trypsin include TrypLE^{™} Express Enzyme (1×), and no phenol red (registered trademark, Thermo Fisher Scientific, Inc.).

The treatment temperature of the enzyme treatment may be any temperature as long as the enzyme to be used can exhibit enzyme activity. Since the effect on the cells in the cell fragment of the living tissue is suppressed, the treatment temperature of the enzyme treatment is preferably 30°C or higher and 40°C or lower, and more preferably 37°C. In addition, the treatment time of the enzyme treatment is not particularly limited, and can be set to, for example, 5 minutes or more and 90 minutes or less, and is preferably 10 minutes or more and 60 minutes or less.

It is preferable to measure the number of cells before seeding the single-celled cancer organoids on the top surface or inside of the cell structure, and it is particularly preferable to measure the number of living cells. The measurement of the number of cells and the measurement of the number of living cells can be performed by a common method. For example, the measurement of the number of living cells includes a staining method using trypan blue.

The cancer organoid may be washed with a buffer solution or a culture medium before the enzyme treatment. As the buffer solution used for washing, a phosphate buffer solution, an acetate buffer solution, a citrate buffer solution, a borate buffer solution, a tartrate buffer solution, a tris buffer solution, PBS, and the like can be used. In addition, an antibiotic can also be added into the buffer solution or culture medium used for washing. The number of times of washing can be appropriately determined, and is preferably 1 or more and 8 or less. In addition, the washing using the buffer solution or the culture medium may be performed only before the enzyme treatment or before and after the enzyme treatment.

In a case where only specific cells in the cancer organoid are cultured on the top surface or inside of the cell structure, only the cells of a target cell type are sorted from an enzyme-treated product of the cancer organoid, and then the sorted cells are added to the culture medium of the cell structure. Alternatively, the cells are seeded inside the cell structure using the method described in FIG. 1D. The method for sorting specific cells from the enzyme-treated product of the cancer organoid is not particularly limited, and various methods generally used for sorting cells can be appropriately selected and used. For example, specific cells can be sorted by one or more methods selected from the group consisting of flow cytometry, magnetic separation, dielectrophoresis, size fractionation, and density gradient fractionation.

In addition, before culturing the cancer organoids on the top surface or inside of the cell structure, the cells constituting the cancer organoids may be labeled with a fluorescent substance or the like in advance. All the cells in the cancer organoids may be labeled, or only targeted specific cells in the cancer organoids may be labeled. The cell labeling method is not particularly limited, and can be appropriately selected from various labeling methods in the related art. For example, fluorescent labeling and the like using the CellTracker (registered trademark, manufactured by Thermo Fisher Scientific Inc.) or PKH Cell Linker Kit (Sigma-Aldrich) can be suitably used for labeling the cancer cells in the cancer organoids. In addition, in a case where the cancer organoids are single-celled to sort out specific cancer cells, the cancer cell after the sorting treatment may be labeled.

### [Culture Medium]

The culture medium may be a medium containing a growth factor, an artificial inhibitor, or the like as long as it does not contain an extracellular matrix, but it is preferably a general culture medium that is widely used for culturing cultured cell lines. The extracellular matrix is as exemplified in the above-described "Cancer Organoid".

Examples of a general culture medium that is widely used for culturing cultured cell lines include D-MEM, E-MEM, ESC, EBM2, EGM2, MEM α, RPMI-1640, Ham's F-12, and Mccoy's 5A, and media obtained by adding a serum such as calf serum (CS), fetal bovine serum (FBS), and fetal horse serum (HBS) in an amount of about 1% by volume or more and 20% by volume or less (preferably about 10% by volume), and a solution of an antibiotic such as penicillin-streptomycin in an amount of about 0.1% by volume or more and 5% by volume or less (preferably about 1% by volume).

Among these, as the culture medium, D-MEM, ESC, EBM2, EGM2 that does not contain Hidrocortisone as an added factor, RPMI-1640, Ham's F-12, and Mccoy's 5a, and media obtained by adding, to these culture media, the above-described serum in an amount of about 1% by volume or more and 20% by volume or less (preferably about 10% by volume) and a solution of an antibiotic such as penicillin-streptomycin in an amount of about 0.1% by volume or more and 5% by volume or less (preferably about 1% by volume) are preferable, since the cancer organoid in the obtained culture has a ductal structure and can form a vascular network structure in the cell structure. Since Hidrocoritsone inhibits the formation of a vascular network structure in the cell structure, it is preferable that Hydrocoritsone is not contained in the culture medium.

### [Culture Conditions]

The culture conditions can be appropriately set similar to the general culture conditions for animal cells. For example, the culture temperature is preferably about 30°C or higher and 40°C or lower, and most preferably 37°C. In addition, the CO₂ concentration is preferably about 1% by volume or more and 10% by volume or less, and most preferably about 5% by volume. In addition, the cells can also be cultured in an environment in which the O₂ concentration is controlled to be lower than that of the atmospheric air.

The cancer organoid seeded on the top surface or inside of the cell structure preferably has a ductal structure. In a case where the culture is performed using a cancer organoid maintaining the morphology, the cancer organoid in a state closer to the state in the living body can be co-cultured with the cell structure containing stromal cells.

In addition, as shown in Examples described later, the cancer organoids in the culture obtained by the culturing method of the present embodiment are in a state where a ductal structure is maintained in a case where the culture is performed using single-celled cancer organoids, or is in a state where a ductal structure is maintained in a case where the culture is performed using cancer organoids having a ductal structure and maintaining a morphology.

The culture obtained by the culturing method of the present embodiment, which contains cancer organoids having such a ductal structure, is very useful as a sample for evaluating the drug efficacy of a candidate substance for an anti-cancer agent under conditions closer to the conditions in the living body or for selecting an optimal anti-cancer agent for each patient.

### <<Method for Screening Test Substance>>

A method for screening a test substance according to an embodiment of the present invention (hereinafter, sometimes simply referred to as a "screening method of the present embodiment") includes culturing a cell structure including the cancer organoid obtained by the above-described method for culturing a cancer organoid in the presence of a test substance (hereinafter, sometimes referred to as a "culture step in the presence of a test substance") and evaluating an effect of the test substance on the cancer organoid (hereinafter, sometimes referred to as an "evaluation step").

In the screening method of the present embodiment, by using the cell structure including the cancer organoid obtained by the above-described method for culturing a cancer organoid, the drug efficacy of a candidate substance for an anti-cancer agent under conditions closer to conditions in the living body can be evaluated. In addition, in a case where a cancer organoid derived from a cancer patient is used, the screening method of the present embodiment can be used to select an anti-cancer agent that is optimal for the cancer patient.

Next, the steps in the screening method of the present embodiment will be described in detail below.

### <Culture Step in Presence of Test Substance>

In the culture step in the presence of a test substance, a cell structure containing the cancer organoid obtained by the above-described method for culturing a cancer organoid is cultured in the presence of a test substance. The test substance is brought into contact with the cancer organoid, for example, by using a method of mixing and adding the test substance to a medium.

Examples of the test substance include a natural compound library, a synthetic compound library, an existing drug library, and a metabolite library. The existing drugs include known anti-cancer agents such as AMG 510 and cetuximab. In addition, a new drug may be used as the test substance. The test substance may be used alone or in combination of two or more types thereof. The amount of the test substance to be administered can be appropriately selected from the type, amount, and the like of the cancer cell constituting the cancer organoid.

### <Evaluation Step>

In the evaluation step, the effect of the test substance on the cancer organoid is evaluated.

In the evaluation step, the effect of the test substance on the cancer organoid can be tested or evaluated by Western blotting, ELISA, immunostaining, or the like.

In addition, in the evaluation step, in a case where single-celled cancer organoids are used, the rate of remaining cancer cells can be calculated by measuring the number of living cancer cells at the time of seeding and measuring the number of living cancer cells after contact with the test substance. That is, the evaluation step may be a step of calculating a ratio of the number of living cancer cells after the contact with the test substance to the number of living cancer cells at the time of seeding.

The method for measuring the number of living cancer cells will be described in detail. The number of living cancer cells can be evaluated using a signal that correlates with the living cancer cells or the abundance thereof. It is sufficient that the number of living cancer cells at the evaluation time point can be measured, and it is not always necessary to measure the number of cells in a living state. For example, the cancer cell can be labeled to be distinguished from other cells, and the signal from the label can be investigated as an indicator. For example, after the cancer cells are fluorescently labeled, the living cancer cells in the cell structure can be directly counted by determining whether the cells are alive or dead. In this case, an image analysis technology can also be used. The determination of the viability of the cells can be performed by a known method for determining the viability of cells, such as trypan blue staining or propidium iodide (PI) staining. The fluorescent labeling of the cancer cell can be performed by a known method such as an immunostaining method using, for example, an antibody against a substance specifically expressed on the cell surface of the cancer cell as a primary antibody and a fluorescent-labeled secondary antibody that specifically binds to the primary antibody. The determination of the viability of the cell and the measurement of the number of living cells may be performed in a state of the cell structure, or may be performed in a state in which the cell structure is destroyed at a single cell level. For example, after destroying the three-dimensional structure of the cell structure after labeling cancer cells and dead cells, only the cancer cells that have been alive at the evaluation time point can be directly counted by fluorescence activated cell sorting (FACS) or the like using the labeling as an indicator.

Examples of the labeling substance for labeling cancer cells include a fluorescent pigment, fluorescent beads, quantum dots, biotin, antibody, antigen, energy absorbing material, radioisotopes, chemical illumination, and enzymes, and the labeling substance is not limited thereto. Among these, a fluorescent pigment is preferably used as the labeling substance. More specific examples of the fluorescent pigment include carboxyfluorescein (FAM), 6-carboxy-4',5'-dichloro-2',7'-dimethoxy fluorescein (JOE), fluorescein isothiocyanate (FITC), tetrachloro-fluorescein (TET), 5'-hexachloro-fluorescein-CE phosphoramidite (HEX), Cy3, Cy5, Alexa568, Alexa647, PKH26, and PKH67GL.

In addition, in the evaluation step, as a control group, the rate of remaining cancer cells in a case where a cell structure containing cancer organoids is cultured in the absence of the test substance may be calculated. That is, the evaluation step may be a step of comparing the rate of remaining cancer cells in the case where the cell structure containing the cancer organoids is cultured in the absence of the test substance with the rate of remaining cancer cells in the case where the cell structure containing the cancer organoids in the presence of the test substance.

In this case, in a case where the rate of remaining cancer cells in the test substance-present group is lower than the rate of remaining cancer cells in the test substance-absent group, it can be determined that the cancer organoids have drug sensitivity to the test substance, and the test substance is effective for the cancer organoids.

On the other hand, in a case where the rate of remaining cancer cells in the test substance-present group is not changed or is increased as compared with the rate of remaining cancer cells in the test substance-absent group, it can be determined that the cancer cells have acquired drug resistance to the test substance and the test substance has no effect on the cancer organoids.

### Examples

Hereinafter, the above-described embodiments of the present invention will be specifically described with reference to Examples. However, the present invention is not limited to the following Examples, and a number of modifications can be made by those with ordinary knowledge in the field of art without departing from the gist of the present invention.

### <Materials>

### 1. Culture Cell and Cancer Organoid

The cell lines and cancer organoids used, and media used for culturing these are shown in Table 1 below. As a patient-derived cancer organoid HT191, a cancer organoid derived from a patent of colorectal cancer (KRAS G12C mutation positive), constructed based on the method described in Reference Document 2 (Japanese Patent No. 6639634), was used.

**[Table 1]**

| Type | Manufacturer name | Medium |
|---|---|---|
| Human umbilical vein endothelial cell (HUVEC) | Lonza | EGM^{™}-2MV BulletKiT^{™} |
| Normal Human Dermal Fibroblast (NHDF) | Lonza | D-MEM + FBS (10 v/v%) |
| Patient-derived cancer organoid | - | IntestiCult Organoid Growth Medium (Human) |
| (PDO) | | |
| (HT191) | | |

### 2. Reagents and the Like

The reagents, consumables such as a culture vessel, and antibodies used are shown in each of Tables 2 to 4 below.

**[Table 2]**

| Reagent name | Item number | Manufacturer name |
|---|---|---|
| D-MEM (high glucose) | 043-30085 | FUJIFILM Wako Pure Chemical Corporation |
| EGM^{™}.-2MV BulletKiT^{™} | CC-3202 | Lonza |
| IntestiCult Organoid Growth Medium (Human) | ST-06010 | STEMCELL Technologies |
| Fetel Bovine Serum (FBS) | 10437028 | Gibco |
| Penicillin-streptomycin solution (P/S) | 168-23191 | FUJIFILM Wako Pure Chemical Corporation |
| Trypsin-EDTA | 25200072 | Invitrogen |
| TrypLE^{™} Express Enzyme (1x), no phenol red | 12604013 | Invitrogen |
| Trypan Blue | 207-17081 | Invitrogen |
| Heparin sodium salt (derived from porcine intestinal mucosa) | H3393-100KU | Sigma-Aldrich |
| Collagen (type I, derived from bovine dermis, acid extracted) | ASC-1-100- | Nippi. Inc. |
| Bovine plasma fibronectin | - | Sigma-Aldrich |
| Acetic acid | 017-00256 | FUJIFILM Wako Pure Chemical Corporation |
| UtraPure^{™} 1 M Tris-HCl Buffer, pH 7.5 | 15567027 | Invitrogen |
| Sterilized water (CultureSure (registered trademark)) | 039-24155 | FUJIFILM Wako Pure Chemical Corporation |
| PBS(-) | 166-23555 | FUJIFILM Wako Pure Chemical Corporation |
| 10 v/v% neutral buffered formalin | 133-10311 | FUJIFILM Wako Pure Chemical Corporation |
| Matrigel | 354234 | Corning |
| Bovine serum albumin (BSA) | - | Thermo Fisher Scientific |
| Goat anti-Rabbit IgG (H + L) Highly Cross-Adsorbed Secondary Antibody, Alexa Flour 594 | A-11037 | Invitrogen |
| Goat anti-Rabbit IgG (H + L) Highly Cross-Adsorbed Secondary Antibody, Alexa Flour 647 | A-21245 | Invitrogen |
| Goat anti-Mouse IgG (H + L) Highly Cross-Adsorbed Secondary Antibody, Alexa Flour 594 | A-11032 | Invitrogen |
| Goat anti-Mouse IgG (H + L) Highly Cross-Adsorbed Secondary Antibody, Alexa Flour 647 | A-21236 | Invitrogen |

**[Table 3]**

| Reagent name | Item number | Manufacturer name |
|---|---|---|
| 200 µL Graduated PP rack yellow | 1201-705YS | Watson |
| Pipette 50 mL, graduated 1 mL, with cotton plug (2-way scale) | 357550 | Falcon |
| Pipette 25 mL, graduated 1 mL, with cotton plug (2-way scale) | 357525 | Falcon |
| Pipette 10 mL, graduated 1 mL, with cotton plug (2-way scale) | 357551 | Falcon |
| Pathstool pipette | 63B95 | CSG |
| 1.5 mL flat gate tube | 131-515CS | Watson |
| Cell culture dish 90 mm (deep type) | MS-13900 | Sumitomo Bakelite Co., Ltd. |
| Square Dish Cell Cultivated Surface Treatment Processed | 431110 | Corning |
| Polyester (PET) Membrane Transwell-Clear Inserts 24 wells | 3470 | Corning |
| Costar (registered trademark) 24-well Clear TC-Treated Multiple Well Plates | 3524 | Corning |
| Countess Cell Counting Chamber Slides | C10312 | Thermo Fisher Scientific |

**[Table 4]**

| Antibody | Manufacturer name | Host | Clone | Dilution ratio | Secondary antibody |
|---|---|---|---|---|---|
| CD31 | DAKO | Mouse | JC70A | 1/50 | 647 |
| EpCAM | CST | Rabbit | D4k8R | 1/100 | 594 |

### 3. Measurement Device

The number of cells in the cell suspension was counted using an automatic cell counter Countess (registered trademark) II FL (Thermo Fisher Scientific, Inc.). For image capture and image analysis of fluorescence immunostaining, an Operetta CLS^{™} high-content imaging system (PerkinElmer, Inc.) was used.

### [Example 1]

The cancer organoids were seeded and cultured on the top surface or inside of the cell structure as they were or after being prepared as single cells, and the proliferation of the cancer organoids was confirmed.

In addition, D-MEM or IntestiCult Organoid Growth Medium was used for co-culture of the PDO and the cell structure described later. D-MEM was used by adding FBS to a concentration of 10 (v/v)% and a penicillin-streptomycin solution to a concentration of 1 (v/v)%. All cells were aseptically handled in a safety cabinet and cultured in an incubator at 37°C in a 5 (v/v)% CO₂ atmosphere.

### 1. Pre-culture of Patient-Derived Cancer Organoid (PDO)

In a case of subculturing the cells while maintaining the morphology of the PDOs, a medium was added, and pipetting was performed to physically disperse Matrigel and the PDOs.

In a case of single-celled PDOs, the PDOs were washed with PBS and then treated with TrypLE^{™} Express Enzyme (1×), and no phenol red at 37°C for 15 minutes to disperse the PDOs. Thereafter, the reaction was stopped in the medium, and the single-celled PDOs was recovered.

Thereafter, in a case of replating the PDOs, the cells were suspended in Matrigel while the tube of the cell pellet was cooled on ice. In this case, the suspension was carried out quickly and carefully so that air bubbles were not generated. 20 µL of a cell suspension gel was applied and spread to the center of a 24-well plate cooled on ice using the tip end of a tip for applying the gel to about half the diameter of the well. At this time, the gel was carefully applied so that the gel did not touch the edge of the well. The plate was incubated upside down to solidify the gel, and then 500 µL/well of a medium (IntestiCult Organoid Growth Medium) was added. In a case of 48 wells, 250 µL/well of a medium (IntestiCult Organoid Growth Medium) was added.

### 2. Preparation of Cell Structure

A cell structure was prepared using a heparin method according to a previously reported procedure.

Specifically, first, a cell coat solution was prepared by mixing equal amounts of a 5 mM acetic acid solution containing 0.2 mg/mL of collagen and a 100 mM Tris-HCl buffer (pH 7.4) containing 0.2 mg/mL of heparin, and diluting the mixture to a final concentration of 0.1 mg/mL.

In a case where PDOs were cultured on the top surface of the cell structure, the cell suspension was mixed so that NHDF was 2 × 10⁶ cells/well (for 20 layers) and the HUVEC was 3 × 10⁴ cells/well (1.5% of NHDF) and centrifuged, and then the supernatant was removed. On the other hand, in a case where the PDOs were cultured inside the cell structure, the cell suspension was mixed so that NHDF was 1 × 10⁶ cells/well (for 10 layers) and HUVEC was 1.5 × 10⁴ cells/well (1.5% of NHDF) and centrifuged, and then the supernatant was removed.

Subsequently, the cells were suspended in the cell coat solution and centrifuged, and then the supernatant was removed. Thereafter, the cells were suspended in the medium and seeded in a culture insert for a 24-well plate. The insert was coated with a 0.1 mg/mL fibronectin solution for 30 minutes or more in advance, and the solution was removed. 1 mL of D-MEM medium was dispensed into the wells of the 24-well plate, and 300 µL of the cell suspension was dispensed into the insert, then the plate was centrifuged to precipitate the cells, and the cells were allowed to stand in an incubator for 2 hours. Then, 1 mL of a medium was added thereto, and the cells were incubated at 37°C for 1 day.

### 3. Co-culture of PDO and Cell Structure

Next, the PDOs maintaining the morphology or single-celled PDOs were dispersed in the method described in "1." above, and the number of cells was measured. Then, the cells were seeded to the cell structure so as to have any seeding number (1 × 10⁴ cells/well for the single-cell PDO, and 200 ± 20 cells/well for the PDO maintaining the morphology).

Specifically, in a case where the PDOs was cultured on the top surface of the cell structure, the medium was added after the PDOs were allowed to stand in an incubator for 2 hours or more after seeding of the PDOs. On the other hand, in a case where the PDOs were cultured inside the cell structure, after seeding the PDOs, a cell suspension containing 1 × 10⁶ cells/well (for 10 layers) of NHDF and 1.5 × 10⁴ cells/well (1.5% of NHDF) of HUVEC was added to the insert after being allowed to stand in the incubator for 2 hours or more, and centrifugation was carried out at 400 × g for 1 minute. After allowing the cells to stand in the incubator for 2 hours or more, 1 mL of the medium was added thereto, and the cells were incubated at 37°C.

The day of the start of the preparation of the cell structure was set to Day 0, and after Day 2 (that is, the start date of the co-culture of the PDOs and the cell structure), the tissue was washed twice with PBS and then fixed with 10 v/v% neutral buffered formalin. A sample in which the single-celled PDOs were seeded on the top surface of the cell structure and cultured was fixed on Days 2, 6, 9, and 14. On Day 2, the cells were fixed 8 hours after the seeding of the PDOs. A sample in which the single-celled PDOs were seeded and cultured inside the cell structure was fixed on Days 4, 7, and 9. One hour after the start of the fixation, formalin was removed, the cells were washed twice with PBS, and then the cells were immersed in PBS.

For the sample to be continuously cultured, the medium was replaced using any medium at a timing of once every two days or once every three days.

### 4. Fluorescence Immunostaining (IF)

The fixed tissue was blocked with a PBS solution containing 1 (v/v)% BSA and 0.2 (v/v)% Triton for 1 to 2 hours. Thereafter, a primary antibody was added at the dilution ratio shown in the above table and treated overnight (O/N). The next day, the primary antibody solution was removed, the cells were washed with PBS, and then a secondary antibody was added at the dilution ratio shown in the above table and treated for 2 hours. The secondary antibody solution was removed, and the cells were washed with PBS. For both the primary antibody and the secondary antibody, the PBS solution containing 1 (v/v)% BSA and 0.2 (v/v)% Triton was used for dilution. In addition, the sample was washed with PBS as necessary, dewatered with 99.5 (v/v)% ethanol, and then subjected to a clearing treatment with a Visikol reagent.

### 5. Image Capturing and Image Analysis

Operetta CLS^{™} was used for capturing images of the stained samples and analyzing the images. The capturing was performed at nine fields of view per well using a 5× objective lens after registration was performed by PreScan using a 1.25× objective lens.

The acquired images were analyzed with image analysis software Harmony. In the analysis, images of four fields of view were tiled in a case of 96 wells, images of nine fields of view were tiled in a case of 24 wells, the images of each plane at different Z positions were combined into one image by Maximum projection, and then the analysis was performed. A region of interest (ROI) was set so as to cover most of the culture insert culture surface, a region (EpCAM positive) having a high fluorescence intensity in the ROI was recognized as a cancer cell, and then the area of the cancer cell was calculated. In addition, the number of cells of Ki-67 included in the EpCAM-positive region was counted.

### 6. Preparation of Section and Immunohistochemical Staining (IHC)

### (1) Preparation of Paraffin Section

The embedding was performed according to a protocol in which a low-toxicity solvent G-Nox (Genostaff Co., Ltd.) was used as a xylene substitute, and paraffin embedding was performed using a paraffin embedding device CT-Pro20 (Genostaff Co., Ltd.) to prepare a block. The block was sliced to a thickness of about 6 µm to prepare a continuous section.

### (2) IHC

The tissue section was deparaffinized with xylene and rehydrated with an ethanol series and a tris-buffered saline (TBS). An antigen was activated by an enzyme treatment (Proteinase K, 5 µg/mL, 37°C, 10 minutes). After washing with PBS at room temperature (about 25°C) three times for 5 minutes, endogenous peroxidase was blocked with 0.3 v/v% hydrogen peroxide-containing methanol for 30 minutes, washed with TBS at room temperature (about 25°C) three times for 5 minutes, blocked with G-Block (Genostaff Co., Ltd.), and incubated with an avidin/biotin blocking kit (Vector Laboratories, Inc.). The section was incubated overnight at 4°C with an anti-EpCAM mouse monoclonal antibody (CST).

The section was washed twice with TBS-T at room temperature (about 25°C) for 5 minutes, and washed once with TBS at room temperature (about 25°C) for 5 minutes, biotinylated goat anti-mouse IgG (Vector Laboratories, Inc.) was added thereto, and the cells were incubated at room temperature (about 25°C) for 30 minutes. Then, peroxidase-conjugated streptavidin (Nichirei Corporation) was added thereto for 5 minutes. The section was washed twice with TBS-T at room temperature (about 25°C) for 5 minutes, and then washed once with TBS at room temperature (about 25°C) for 5 minutes, and then the peroxidase activity was visualized with diaminobenzidine. The section was counterstained with Mayer's Hematoxylin (MUTO PURE CHEMICALS CO., LTD.), dewatered and then mounted with Malinol (MUTO PURE CHEMICALS CO., LTD.).

### 7. Results and Consideration

### (1) Co-culture of Single-Celled PDO on Top Surface of Cell Structure

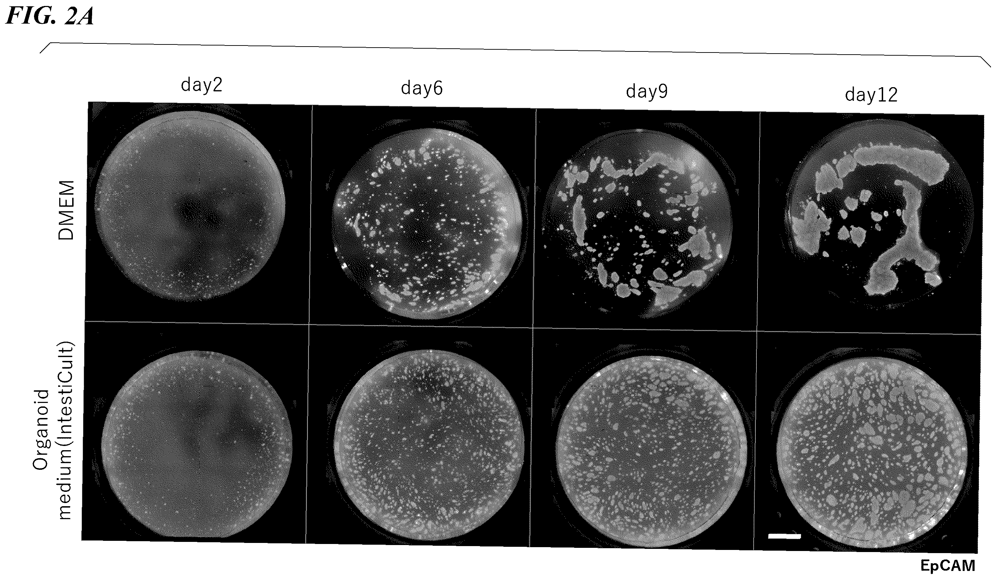

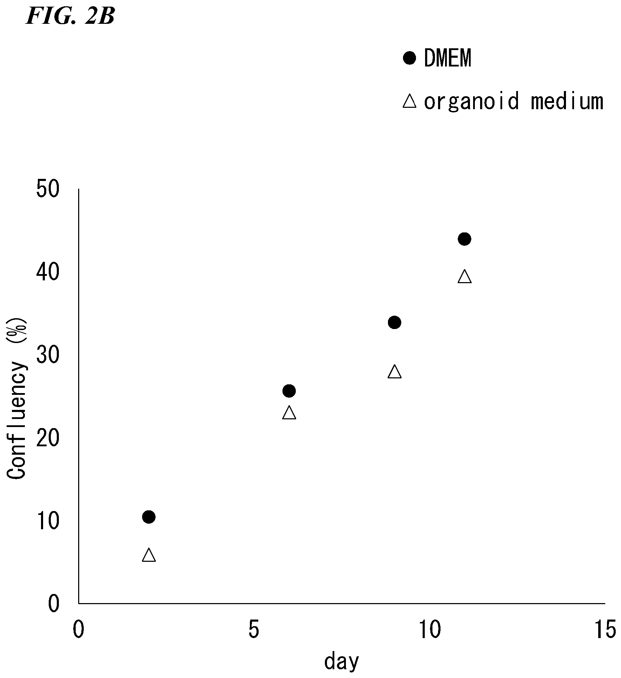

FIG. 2A (fluorescent immunostaining images of PDOs) and FIG. 2B (a graph of confluency (%) of PDOs) show the results of confirming the proliferation when the PDOs dispersed as single cells are seeded on the top surface of the cell structure. The scale bar in FIG. 2A is 1 mm.

As shown in FIGS. 2A and 2B, it was found that, in a case where PDOs dispersed as single cells were seeded on the top surface of the cell structure in a dispersed state as single cells, the cells proliferated over time even in a case of using any medium of D-MEM and an organoid medium. Focusing on the difference in the medium, in a case of culturing in the D-MEM, the PDOs were accumulated and cells proliferated, whereas in a case of culturing in the organoid medium, the PDOs remained at the positions at the time of seeding and proliferated.

### (2) Co-culture of Single-Celled PDO Inside Cell Structure

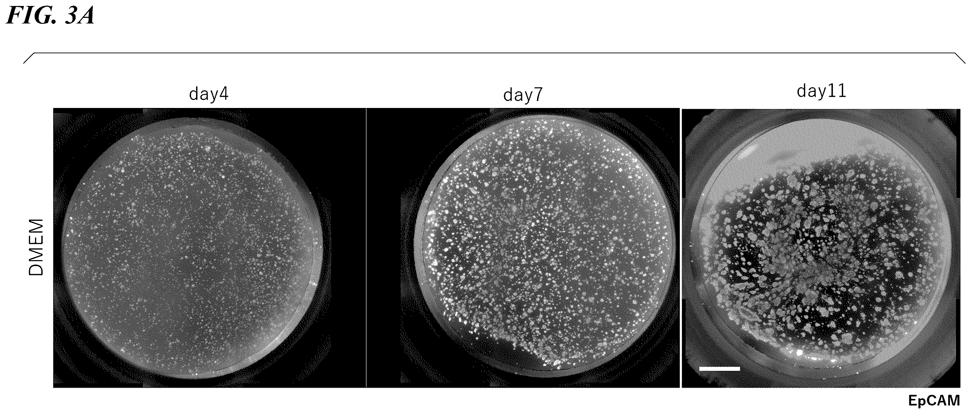

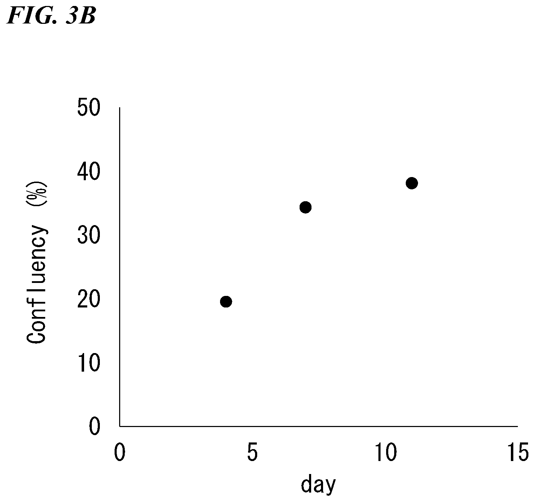

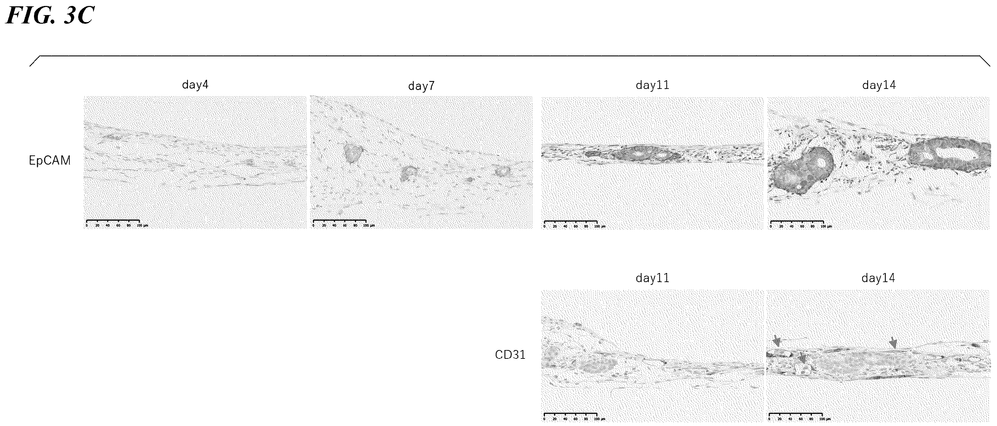

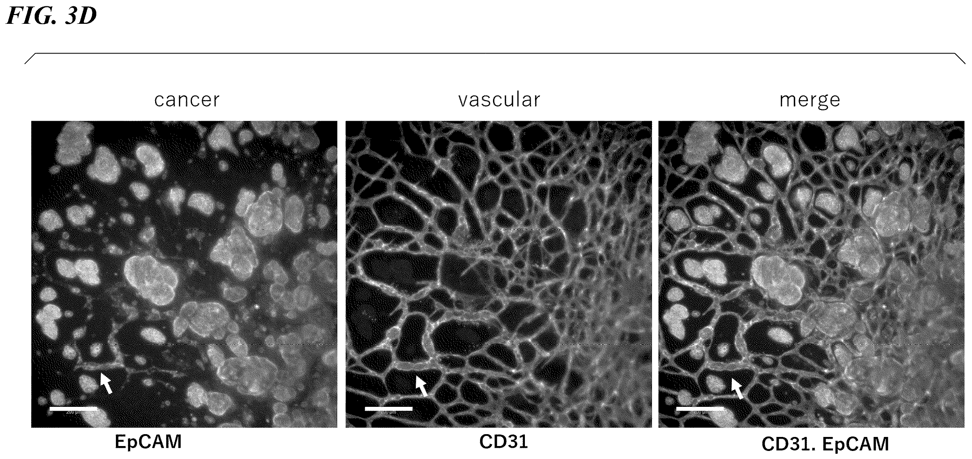

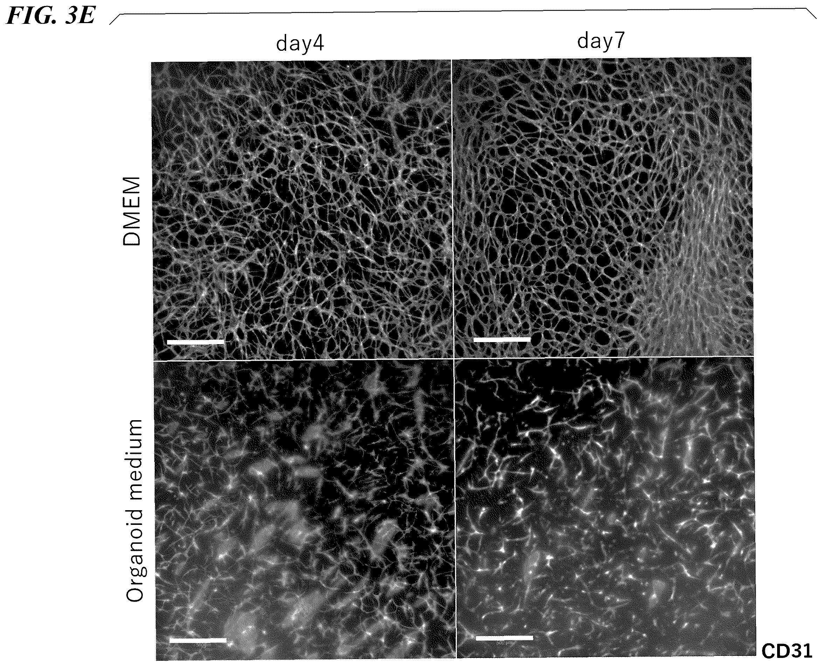

The results of confirming the proliferation when the PDOs dispersed as single cells are seeded inside the cell structure is shown in FIG. 3A (fluorescent immunostaining images of PDOs), FIG. 3B (a graph of confluency (%) of PDOs), FIG. 3C (immunostaining images of PDOs and vascular network structure in sections), FIG. 3D (fluorescent immunostaining images of PDOs and vascular network structure in sections), and FIG. 3E (fluorescent immunostaining images of vascular network structure in sections). The scale bar in FIG. 3A is 1 mm. The arrow in FIG. 3C indicates the PDO that is infiltrated into the blood vessel-like vascular network structure. The scale bar in FIG. 3D is 200 µm. The arrow in FIG. 3D indicates the PDO that is infiltrated into the blood vessel-like vascular network structure.

As shown in FIGS. 3A and 3B, it was found that the PDOs proliferated over time even in a case where the PDOs dispersed as single cells were seeded inside the cell structure.

In addition, from the immunostaining images of the sections using the anti-EpCAM antibody in FIG. 3C, it was confirmed that the PDOs dispersed as single cells formed a ductal structure after Day 7 under the condition that the PDOs were seeded inside the cell structure.

Furthermore, from the immunostaining images of the section using the anti-CD31 antibody in FIGS. 3C and 3D, it was confirmed that the PDOs infiltrated into part of the blood vessel-like vascular network structure. In addition, the observation was made that the blood vessel-like vascular network structure was enlarged in morphology as compared with a normal state, and the blood vessel-like luminal structure was attracted to the PDOs.

As shown in FIG. 3E, it was found that in the organoid medium, the blood vessel-like vascular network structure could not be formed due to the aggregation of the endothelial cells. From this, it was presumed that D-MEM, rather than an organoid medium, was suitable as a medium in order to evaluate the interaction between the blood vessel-like vascular network structure and the PDOs.

### (3) Co-culture of PDO Maintaining Morphology on Top Surface of Cell Structure

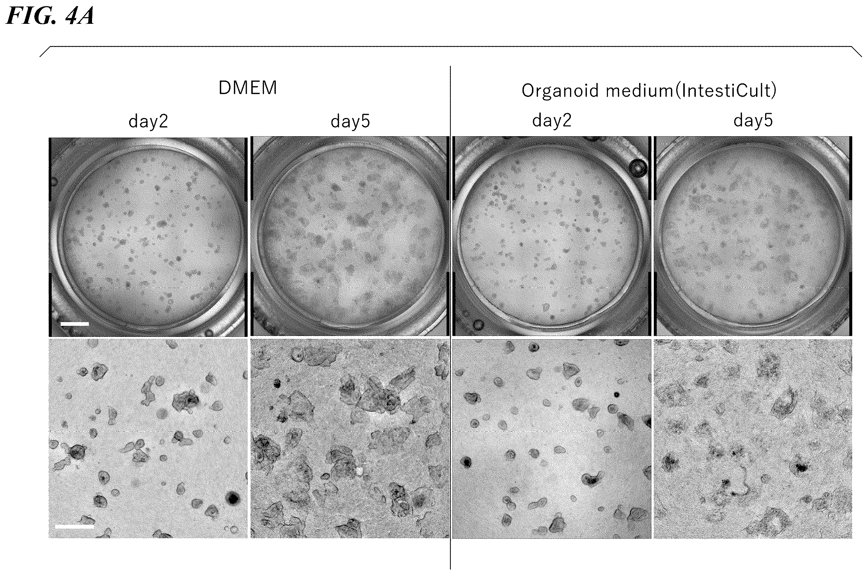

FIGS. 4A (bright field images) and 4B (immunostaining images of PDOs in sections) show the results of confirming the proliferation in a case where the PDOs maintaining the morphology are seeded on the top surface of the cell structure. In FIG. 4A, the scale bar in the upper part is 1 mm, and the scale bar in the lower part is 500 µm.

As shown in FIG. 4A, it was confirmed that PDOs were attached to the top surface of the cell structure and the three-dimensional structure of the PDO was slightly flattened as the culture time was elapsed. Although it is difficult to perform quantitative evaluation due to the bright field, it was found that the cells themselves proliferated over time even in a case of using any medium of D-MEM and an organoid medium.

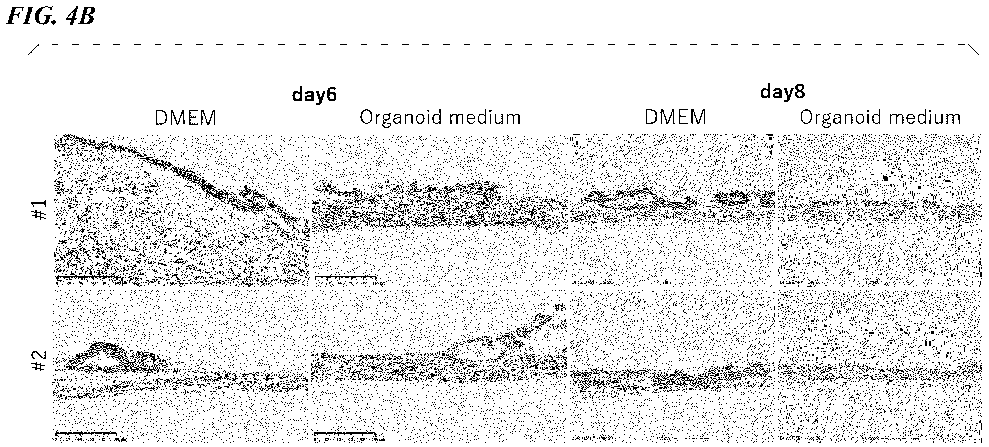

As shown in FIG. 4B, it was found that, in a case of culturing in D-MEM, the PDOs maintained the morphology of the ductal structure, whereas in the case of culturing in the organoid medium, the ductal structure disappeared and the PDOs were present on the top surface of the cell structure in a flat morphology.

From the above, the following became clear.

In a case where the single-celled PDOs were cultured on the top surface or inside of the cell structure, the PDOs proliferated even in a case of using any medium of D-MEM and an organoid medium. In addition, it was confirmed that the PDOs formed a ductal structure either on the top surface or inside of the cell structure in a case where D-MEM was used as a medium. In addition, the infiltration into the blood vessel-like vascular network structure was also observed.

In a case where the PDOs maintaining the morphology were cultured on the top surface of the cell structure, the PDOs proliferated even in a case of using any medium of D-MEM and an organoid medium. In addition, it was found that, in a case where D-MEM was used as a medium, the ductal structure could be maintained, whereas in a case where an organoid medium was used as a medium, the ductal structure of the PDOs could not be maintained.

Furthermore, it was found that in a case where D-MEM was used as a medium, the cell structure could form a vascular network structure, whereas in a case where an organoid medium is used as a medium, the cell structure could not form a vascular network structure.

### [Example 2]

From the results of Example 1, it was found that, under the condition in which the PDOs maintaining the morphology were seeded, it was difficult to seed any number of cells in the cell structure by measuring the number of cells, but under the condition in which the single-celled PDOs were seeded, the cells could be seeded by measuring the number of cells, which was suitable for the drug efficacy evaluation. Therefore, a drug efficacy evaluation test was performed under the condition in which the single-celled PDOs were seeded on the top surface of the cell structure.

Since the used PDOs had a G12C mutation in the KRAS gene, as drugs, AMG 510 (concentration in medium: 0, 0.1, 1, and 10 µM), a G12C inhibitor, as a single agent, and cetuximab(concentration in medium: 50 µg/mL), an EGFR inhibitor, reported to have a combination effect with the G12C inhibitor, were used in combination. D-MEM was used as a medium.

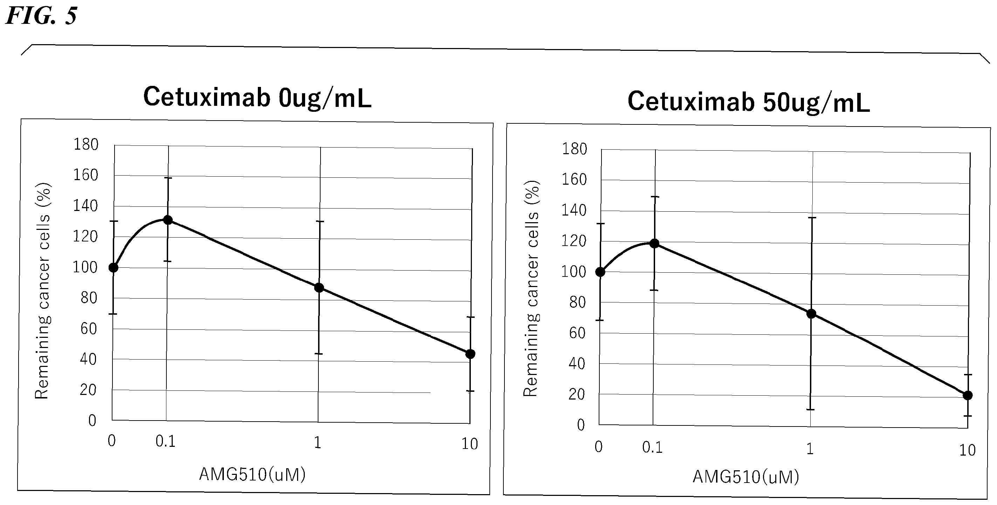

First, using the same method as in Example 1, the day of the start of the construction of the cell structure was set to Day 0, the medium was replaced on Day 1, single-celled PDOs (5 × 10³ cells/well; 96-well plate) were seeded on the top surface of the cell structure on Day 2. Next, the drug was added on Day 5, the cells were fixed on Day 8, and the number of living cancer cells was measured. Next, the ratio of the number of cells remaining under each condition to the number of cells under the condition in which the concentration of AMG 510 was 0 µM and the concentration of cetuximab was 0 µg/mL was calculated as the rate (%) of remaining cancer cells. The results are shown in FIG. 5. In FIG. 5, the horizontal axis represents the concentration of AMG 510 in the medium, and the vertical axis represents the rate (%) of remaining cancer cells.

As shown in FIG. 5, in both the use of AMG 510 as a single agent and the use of AMG 510 and cetuximab in combination, a decrease in the number of cancer cells was confirmed in a drug concentration-dependent manner of AMG 510.

From the above results, the drug efficacy of the drug expected from the effect estimated from the gene mutation of the PDOs could be actually confirmed.

### [Example 3]

Organoids different from those in Example 1 were seeded on the top surface or inside of the cell structure and cultured, and the proliferation of the cancer organoids was confirmed. In addition, the medium having an effect on the formation of a vascular network was examined.

### <Materials>

### 1. Culture Cell and Cancer Organoid

Table 5 shows the cell lines and the cancer organoids used. The patient-derived PDO HCT261T is a PDO of a primary lesion derived from a patient with colorectal cancer. The patient-derived PDO HCT26 3LM is a colonic PDO of a metastatic lesion derived from a patient with colorectal cancer. The patient-derived PDO HCT261T and the patient-derived PDO HCT26 3LM established based on the method described in Reference Document 1 were used.

**[Table 5]**

| Type | Source | Medium |
|---|---|---|
| Patient-derived PDO (HCT26 1T) primary lesion | Japanese Foundation for Cancer Research | IntestiCult Organoid Growth Medium |
| Patient-derived PDO (HCT26 3LT) metastatic lesion | Japanese Foundation for Cancer Research | IntestiCult Organoid Growth Medium |

HUVEC and NHDF used for constructing the cell structure are the same as those in Example 1.

### 2. Reagents and Measuring Device

The reagents used are shown in Table 6. Consumables such as a culture vessel, antibodies, and a measuring device are the same as in Example 1.

**[Table 6]**

| Reagent name | Item number | Manufacturer name |
|---|---|---|
| DMEM (high glucose) | 043-30085 | FUJIFILM Wako Pure Chemical Corporation |
| EGM^{™}-2 MV BulletKit^{™} | CC-3202 | Lonza |
| IntestiCult Organoid Growth Medium (Human) | ST-06010 | STEMCELL Technologies |
| RPMI-1640 | 189-02025 | FUJIFILM Wako Pure Chemical Corporation |
| McCoy 5A | 16600108 | Thermo Fisher Scientific |
| Ham's F-12 | 087-08335 | FUJIFILM Wako Pure Chemical Corporation |
| StemPro hESC | A1000701 | Thermo Fisher Scientific |
| Fetal bovine serum (FBS) | 10437028 | Gibco |
| Penicillin-streptomycin solution (P/S) | 168-23191 | FUJIFILM Wako Pure Chemical Corporation |
| Trypsin-EDTA | 25200072 | Invitrogen |
| TrypLE^{™} Express Enzyme (1×), no phenol red | 12604013 | Invitrogen |
| Trypan Blue | 207-17081 | Invitrogen |
| Heparin sodium salt (derived from porcine intestinal mucosa) | H3393-100KU | Sigma-Aldrich |
| Collagen (type I, derived from bovine dermis, acid extracted) | ASC-1-100-100 | Nippi. Inc. |
| Bovine plasma fibronectin | | Sigma-Aldrich |
| Acetic acid | 017-00256 | FUJIFILM Wako Pure Chemical Corporation |
| UltraPure^{™} 1 M Tris-HCI Buffer, pH 7.5 | 15567027 | Invitrogen |
| Sterilized water (CultureSure registered trademark) | 039-24155 | FUJIFILM Wako Pure Chemical Corporation |
| PBS(-) | 166-23555 | FUJIFILM Wako Pure Chemical Corporation |
| 10 v/v% neutral buffered formalin | 133-10311 | FUJIFILM Wako Pure Chemical Corporation |
| Matrigel | 356231 | Corning |
| Bovine serum albumin (BSA) | - | Thermo Fisher Scientific |
| Goat anti-Rabbit IgG (H + L) Highly Cross-Adsorbed Secondary Antibody, Alexa Fluor 594 | A-11037 | Invitrogen |
| Goat anti-Rabbit IgG (H + L) Highly Cross-Adsorbed Secondary Antibody, Alexa Fluor 647 | A-21245 | Invitrogen |
| Goat anti-Mouse IgG (H + L) Highly Cross-Adsorbed Secondary Antibody, Alexa Fluor 594 | A-11032 | Invitrogen |
| Goat anti-Mouse IgG (H + L) Highly Cross-Adsorbed Secondary Antibody, Alexa Fluor 647 | A-21236 | Invitrogen |

Table 7 shows the used medium and the composition of the added factors of the medium.

**[Table 7]**

| Medium | Reagent | Item number | Manufacturer name | Final concentration |
|---|---|---|---|---|
| AIM(+) | AIM-V Medium | 12055091 | gibco | |
| | IL-2 | 200-02 | peprotech | 100 U/mL |
| | Penicillin-streptomycin solution (P/S) | 168-23191 | FUJIFILM Wako Pure Chemical Corporation | 100 U/mL |
| | 1 M HEPES | 15630080 | gibco | 10 mM |
| | 1000 × 2-ME | 21985023 | gibco | 1× (55 mM (1/1000 diluted) |
| | Human Serum AB Male HIV tested | S4190 | Biowest | 0.05 |
| EGM2 | EBM2 medium | CC-3162 | Lonza | Concentration not disclosed |
| | EGF | | | |
| | VEGF | | | |
| | IGF | | | |
| | bFGF | | | |
| | AscP | | | |
| | Hydrocortisone | | | |
| basal medium | advanced DMEM/F12 | 12634010 | Thermo | |
| | Glutamax | 35050061 | Thermo | 2 mM |
| | 1 M HEPES | 15630080 | gibco | 10 mM |
| | Penicillin-streptomycin solution (P/S) | 168-23191 | FUJIFILM Wako Pure Chemical Corporation | 100 U/mL |
| ESC | DMEM/Ham's F-12 + GlutaMAX StemPro 25% BSA | invitrogen | #A1000701 | 0.5× 1.30% |
| | 100 ug/mL bFGF | Nacalai | #100-18B | 6.4 ng/mL |
| | 55 mM 2-Mercapto-etanol | invitrogen | #21985-023 | 80 uM |
| | 20 mM Y-27632 | LC Laboratories | #Y5301 | 8 uM |
| | 100× anti-anti | Nacalai | #02892-54 | 1× |

### <Culture and Image Analysis>

The pre-culture of the patient-derived PDOs and the preparation of the cell structure were performed by the same method as in Example 1. For the co-culture of the PDOs and the cell structure, a method for top surface culture of the cell structure using single-celled PDOs was adopted. In addition, regarding the sample for which the culture was continued, the co-culture of the PDOs and the cell structure was performed by the same method as in Example 1, except that the medium was replaced using each medium at a timing of once every three or four days.

Fluorescence immunostaining, image capturing, and image analysis were performed by the same methods as in Example 1.

### <Results and Consideration>

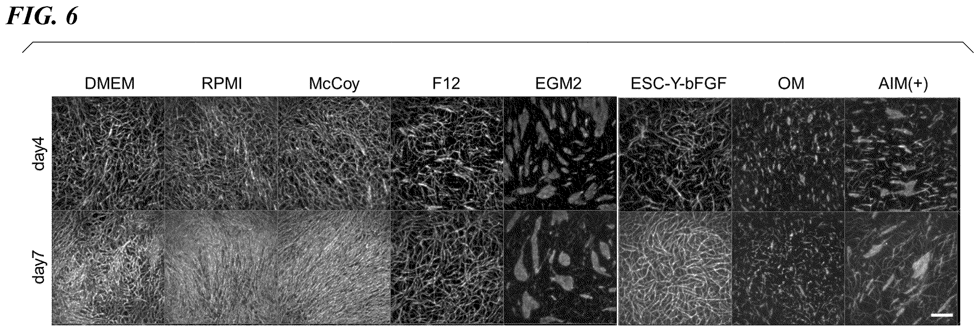

The PDOs and the cell structure were co-cultured using the media D-MEM, InterstiCult Organoid Growth Medium, RPMI-1640, McCoy 5A, and Ham's F12 shown in Table 6, and the media AIM(+), EGM2, and ESC shown in Table 7, and the formation of a blood vessel network was evaluated. FIG. 6 is immunostaining images of the tissues cultured in each medium using an anti-CD31 antibody. The scale bar in FIG. 6 indicates 500 µm. In FIG. 6, DMEM represents D-MEM, OM represents InterstiCult Organoid Growth Medium, RPMI represents RPMI-1640, McCoy represents McCoy 5A, F12 represents Ham's F12, and ESC-Y-bFGF represents ESC.

As a result, the blood vessel network was formed in a case of using D-MEM, RPMI-1640, and McCoy 5A. It was found that when Ham's F12 and ESC were used, the formation of the blood vessel network was observed, although the formation of the blood vessel network was suppressed as compared with a case of using D-MEM. In addition, it was found that in a case of using EGM2, InterstiCult Organoid Growth Medium, and AIM(+), the vascular endothelial cells were aggregated, and the formation of a vascular network itself was suppressed.

In each of EGM2 and AIM(+), the basal medium and the added factors shown in Table 7 were added. Therefore, it was determined whether the factor inhibiting the formation of the blood vessel network was the basal medium or the added factor. Since the InterstiCult Organoid Growth Medium already contained the added factors and the added factors could not be separated, the InterstiCult Organoid Growth Medium was omitted.

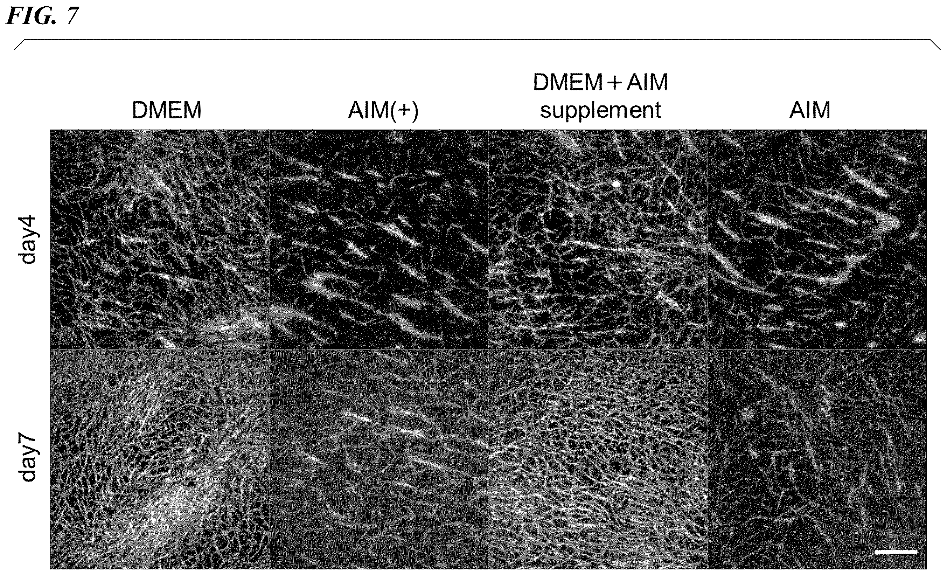

The PDOs and the cell structure were co-cultured using each of D-MEM, AIM(+), D-MEM supplemented with the added factors of AIM(+), and AIM as a medium. The results are shown in FIG. 7. The scale bar in FIG. 7 indicates 500 µm. In FIG. 7, DMEM + AIM supplement indicates that D-MEM supplemented with the added factors of AIM(+) was used.

When D-MEM supplemented with the added factors of AIM(+) was used, a blood vessel network was formed, whereas in the AIM medium to which added factors were not added, the formation of the blood vessel network was suppressed. Therefore, it was suggested that the basal medium of AIM was a factor of the suppression of the blood vessel network.

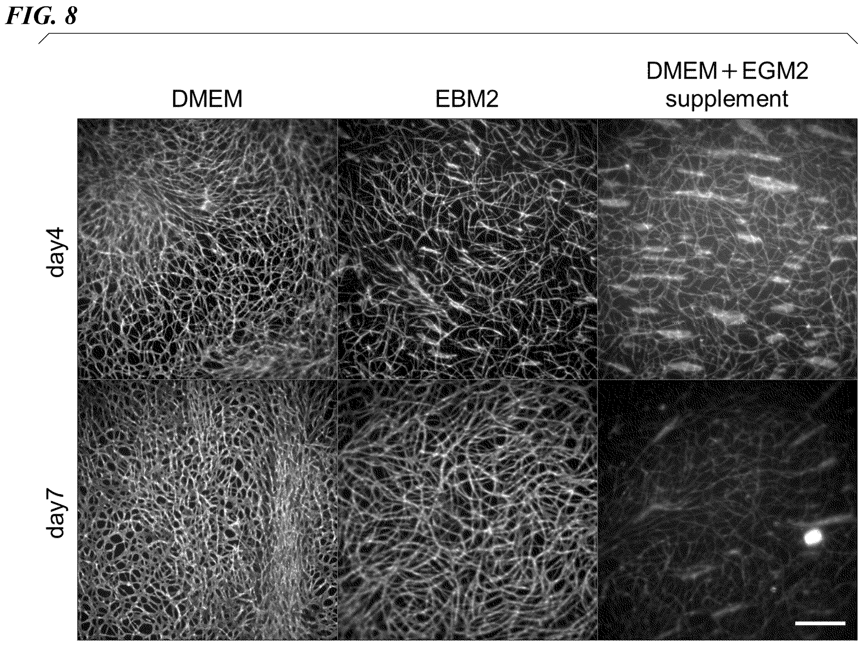

The PDOs and the cell structure were co-cultured using each of D-MEM, EBM2, and D-MEM supplemented with added factors of EGM2 as a medium. The results are shown in FIG. 8. The scale bar in FIG. 8 indicates 500 µm. In FIG. 8, DMEM + EGM2 supplement indicates that D-MEM supplemented with the added factors of EGM2 was used. In EBM2 which is a basal medium of the EGM2 medium, a blood vessel network was formed, whereas in D-MEM supplemented with the added factors of EGM2, the formation of the blood vessel network was suppressed.

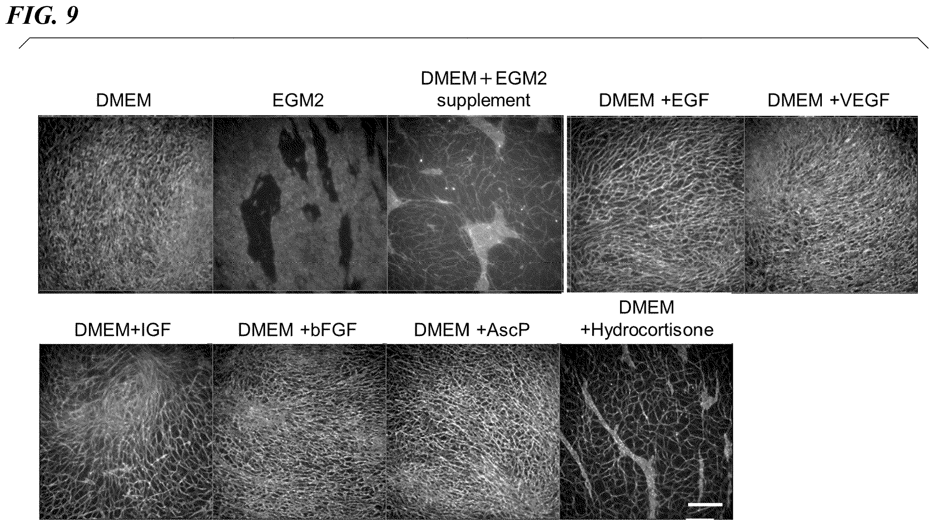

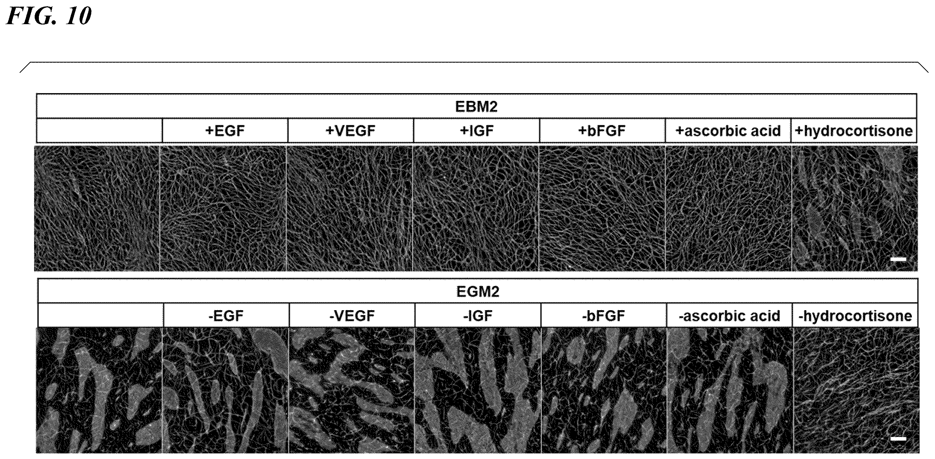

Next, in order to identify the inhibitory factors among the added factors, media obtained by adding each of the added factors of EGM2 shown in Table 7 to D-MEM and EBM2, and media obtained by excluding each of the added factors shown in Table 7 from EGM2 were prepared, and vascular network evaluation was performed. The results are shown in FIGS. 9 and 10. The scale bars in FIGS. 9 and 10 indicate 500 µm. In FIG. 9, DMEM + EGM2 supplement indicates that D-MEM supplemented with all the added factors of EGM2 was used. In FIG. 10, + means that the added factor is included, and - means that the added factor is excluded. As a result, it was found that, from the fact that only in a case where Hydrocortisone was added to each of DMEM and EBM2, the formation of the blood vessel network was suppressed, and in a case where only Hidrocortisone was removed from the EGM2 medium, the blood vessel network was formed, Hidrocortisone was involved in the inhibition of the formation of the blood vessel network.

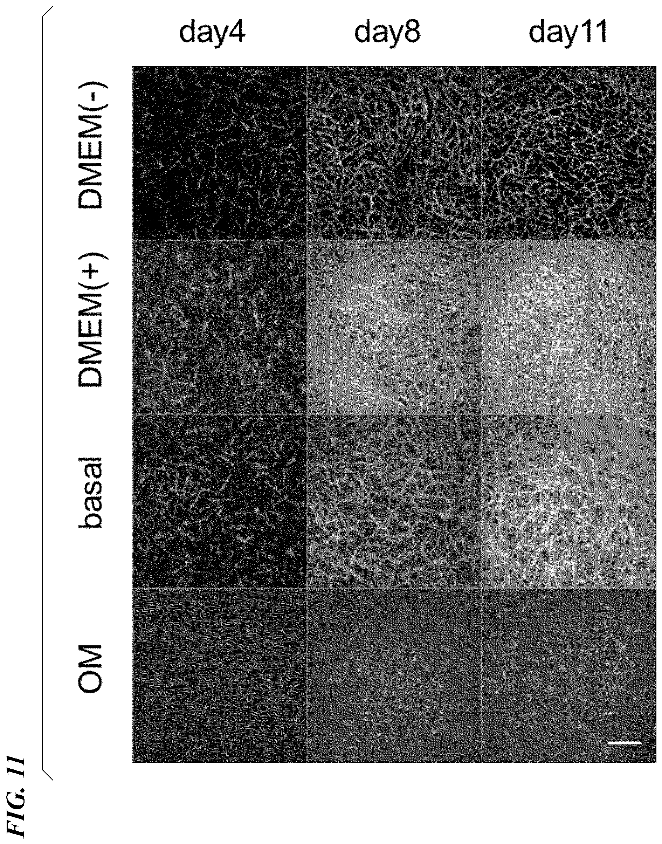

In the InterstiCult Organoid Growth Medium, some added factors are added to the basal medium shown in Table 7. Therefore, the PDOs and the cell structure were co-cultured in the same procedure as in Example 3, using the basal medium as a medium. The results are shown in "basal" in FIG. 11. FIG. 11 also shows the results of co-culture using the InterstiCult Organoid Growth Medium. The scale bar in FIG. 11 indicates 500 µm. As shown in FIG. 11, it was found that the blood vessel network was formed in a case where the basal medium was used. Since the medium obtained by adding various added factors (B27-vitamin A, N-acetylcysteine, Gastrin, EGF, R-spondin 1, Noggin, nicotineamide, A-83-01, and SB202190) to the basal medium is the InterstiCult Organoid Growth Medium, it is considered that these added factors have a blood vessel network formation inhibitory factor.

In addition, in order to investigate the effect of FBS on the formation of blood vessel network, the PDOs and the cell structure were co-cultured in the same procedure as in Example 3 using D-MEM not containing FBS (hereinafter referred to as DMEM(-)) and D-MEM containing FBS (hereinafter referred to as DMEM(+)). The results are shown in "DMEM(+)" and "DMEM(-)" in FIG. 11. In a case where D-MEM was used, there was a difference in the density and amount of the blood vessel network formation depending on the presence or absence of FBS, but the formation of the blood vessel network was confirmed in both cases.

From the above results, it was suggested that the basal medium is a factor in the suppression of the blood vessel network in the AIM medium. In the EGM2 medium, it was suggested that Hydrocortisone as an added factor was a factor inhibiting the blood vessel network.

### [Example 4]

HCT26-1T and HCT26-3LM, which are two types of PDOs different from Example 1, were used to evaluate the proliferation and morphology of cancer. The culture was performed in the same procedure as in Example 3, using DMEM(-), DMEM(+), basal Medium, and InterstiCult Organoid Growth Medium as media. In addition, in the method for culturing PDOs inside the cell structure using single-celled PDOs, the PDOs and the cell structure were co-cultured in the same method as in Example 1, except that the culture was continued and the medium was replaced using each medium at a timing of once every three or four days for the sample to be continuously cultured.

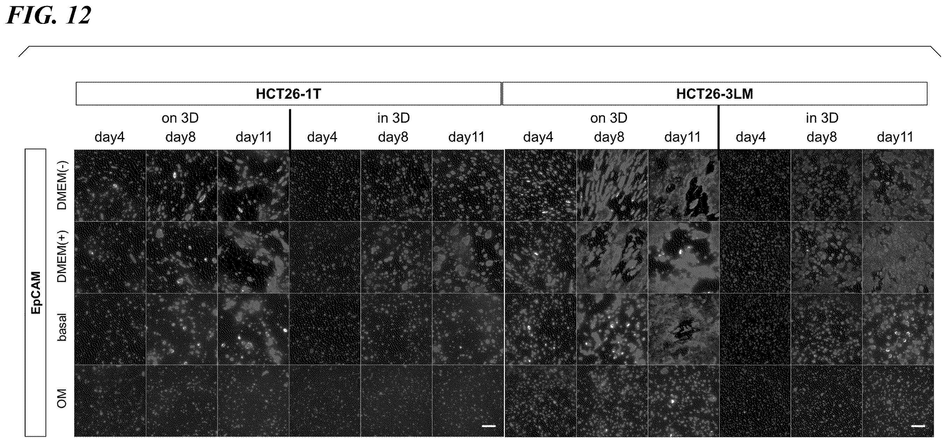

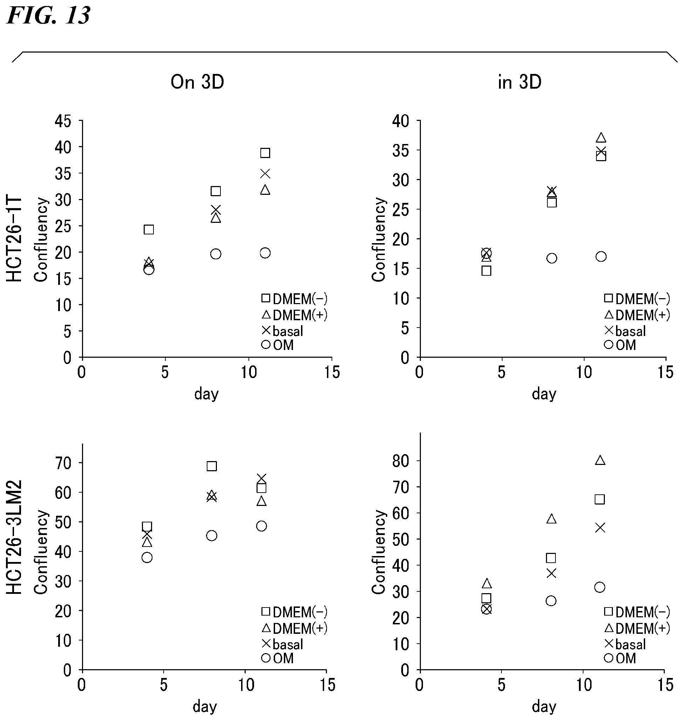

The results are shown in FIG. 12. The scale bar in FIG. 12 indicates 500 µm. In FIG. 12, "on 3D" indicates that the PDOs were cultured on the top surface of the cell structure, and "in 3D" indicates that the PDOs were cultured inside the cell structure. In addition, the confluency (%) of PDOs on days 4, day 8, and day 11 of culture was calculated from the images shown in FIG. 12. FIG. 13 is graphs showing a change over time in the confluency (%) of PDOs in the tissues in which HCT26-1T and HCT26-3LM are cultured in each medium. It was found that HCT26-1T and HCT26-3LM proliferated significantly in the media other than the InterstiCult Organoid Growth Medium even in a case where the PDOs were cultured on the top surface of the cell structure and in a case where the PDOs were cultured inside the cell structure.

Next, in order to confirm the morphology of the PDOs, sections of these tissues were prepared and evaluated.

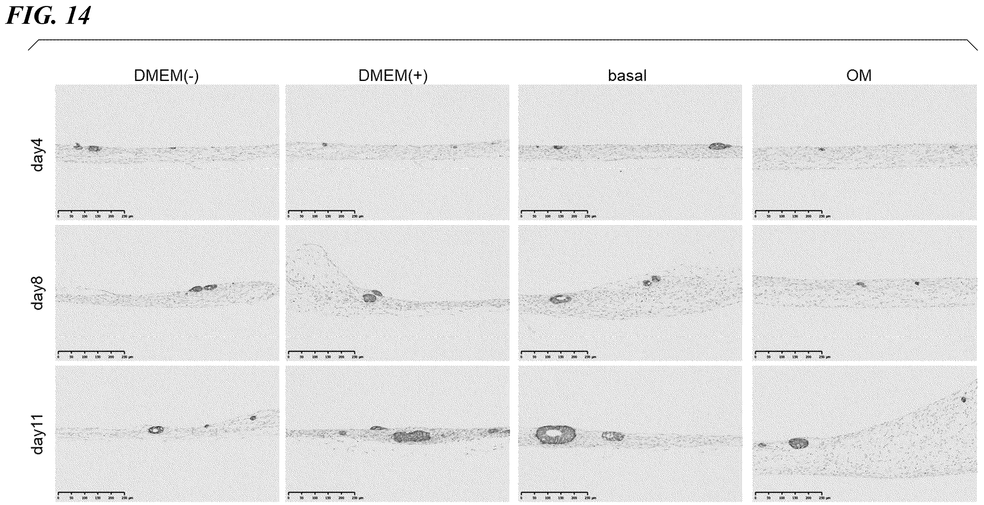

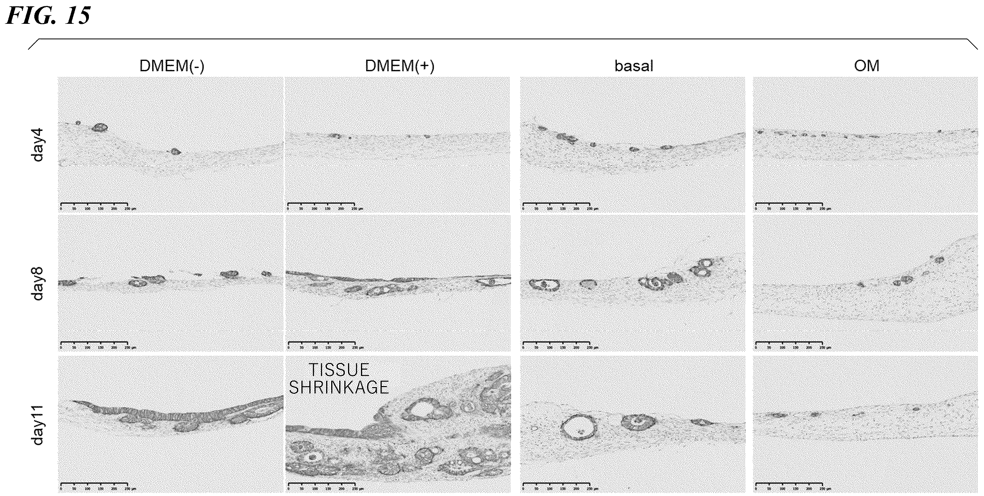

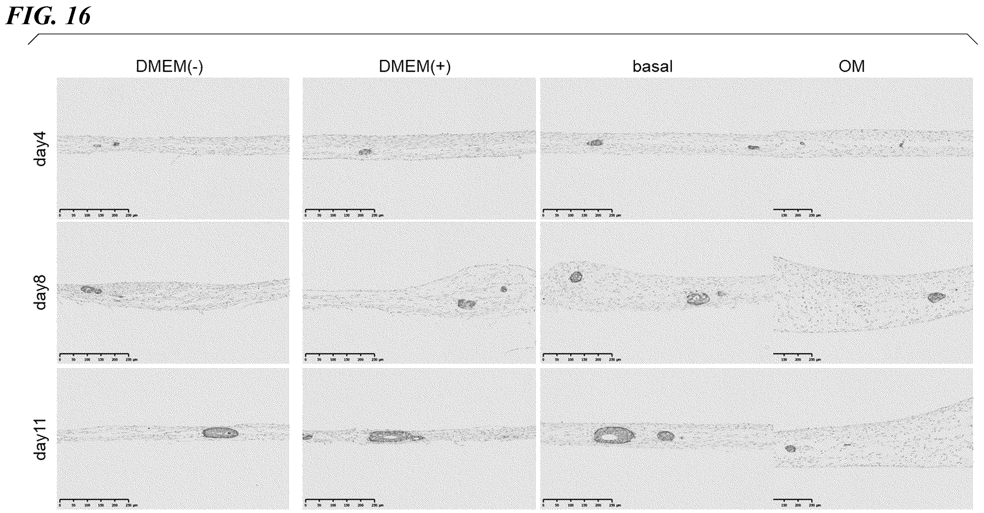

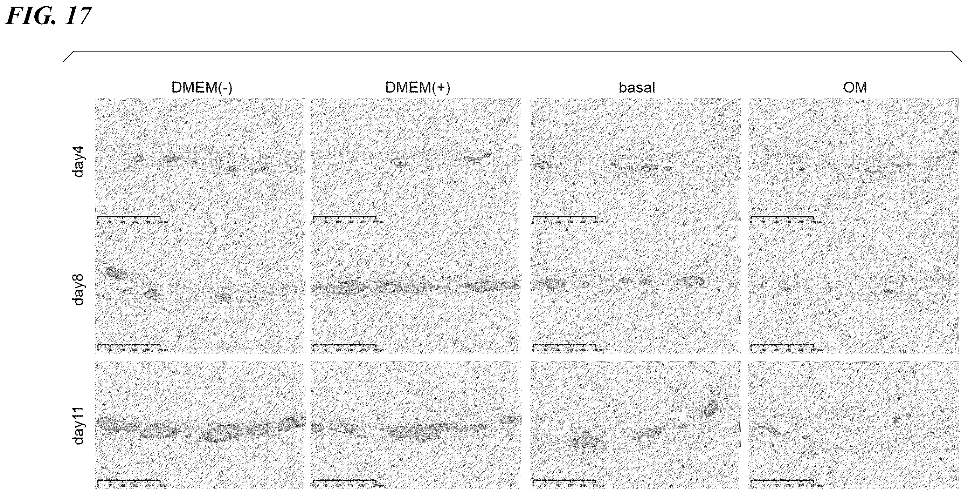

FIG. 14 shows immunostaining images of the PDOs in sections of tissues in a case where HCT26-1T is cultured on the top surface of the cell structure. FIG. 15 shows immunostaining images of the PDOs in sections of tissues in a case where HCT26-3LM is cultured on the top surface of the cell structure. FIG. 16 shows immunostaining images of the PDOs in sections of tissues in a case where HCT26-1T is cultured inside the cell structure. FIG. 17 shows immunostaining images of the PDOs in sections of tissues in a case where HCT26-3LM is cultured inside the cell structure.

The proliferation was poor under the condition using the InterstiCult Organoid Growth Medium. On the other hand, the PDOs having a ductal structure was confirmed even in a case where HCT26-1T and HCT26-3LM were cultured either on the top surface or inside of the cell structure, and even under culture conditions other than the InterstiCult Organoid Growth Medium. In addition, interestingly, even when single-celled HCT26-1T and HCT26-3LM were seeded on the top surface of the cell structure, most of these organoids proliferated inside the cell structure and formed a ductal structure. Although there was a population of PDOs localized on the cellular structure, in many cases, the PDOs were present at a high density. In a case where the PDOs had a high density, a ductal structure was not formed and a columnar epithelium-like structure was formed.

From the above results, it was confirmed that in HCT26 1T and HCT26 3LM which were PDOs established from clinical samples, the formation of a blood vessel network and the proliferation of the PDOs occurred in the D-MEM and the basal medium, but the formation of a blood vessel network and the proliferation of the PDOs were suppressed in the InterstiCult Organoid Growth Medium containing additives. In addition, it was confirmed that the PDOs proliferated in the cell structure and formed a ductal structure even in a case where the PDOs were seeded on the top surface of the cell structure.

### INDUSTRIAL APPLICABILITY

According to the method for culturing a cancer organoid of the present embodiment, the cancer organoid can be co-cultured with cells constituting the stroma without using an extracellular matrix while maintaining the properties of the cancer organoid, and cancer cell invasion can be evaluated.

### REFERENCE SIGNS LIST

1 Cancer organoid
1A Single-celled cancer organoid (single cell obtained by dispersing cancer organoid)
2 Cell structure
2A First cell structure
2B Second cell structure
3 Culture vessel

## Claims

1. A method for culturing a cancer organoid, comprising:
culturing the cancer organoid on a top surface or inside of a cell structure in a medium not including an extracellular matrix,
wherein the cell structure includes cells constituting stroma, and has two or more cell layers laminated in a thickness direction.

2. The method for culturing a cancer organoid according to Claim 1,
wherein the culturing includes dispersing the cancer organoid as a single cell and then culturing the cancer organoid on the top surface or inside of the cell structure.

3. The method for culturing a cancer organoid according to Claim 1 or 2,
wherein the culturing includes seeding the cancer organoid on a top surface of a first cell structure having two or more cell layers laminated in the thickness direction, then further seeding a cell suspension containing the cells constituting the stroma to construct a second cell structure on an upper part of the cancer organoid, and culturing the cancer organoid on the inside of the cell structure.

4. The method for culturing a cancer organoid according to Claim 1 or 2,
wherein the medium is a medium consisting of any one or a combination of two or more of D-MEM, ESC, EBM2, EGM2 not containing Hidrocortisone as an added factor, RPMI-1640, Ham's F-12, and Mccoy's 5a.

5. The method for culturing a cancer organoid according to Claim 1 or 2,
wherein the cancer organoid has a ductal structure.

6. The method for culturing a cancer organoid according to Claim 1 or 2,
wherein the cell structure includes one or more types of cells selected from the group consisting of fibroblasts, pericytes, endothelial cells, and immune cells as the cells constituting the stroma.

7. The method for culturing a cancer organoid according to Claim 6,
wherein the endothelial cells are one or more types of cells selected from the group consisting of vascular endothelial cells and lymphatic endothelial cells.

8. The method for culturing a cancer organoid according to Claim 1 or 2,
wherein the cell structure has a vascular network structure.

9. The method for culturing a cancer organoid according to Claim 1 or 2,
wherein in the cell structure, ten or more cell layers are laminated in the thickness direction.

10. A method for screening a test substance, comprising:
culturing a cell structure including the cancer organoid obtained by the method for culturing a cancer organoid according to Claim 1 or 2 in a presence of the test substance; and
evaluating an effect of the test substance on the cancer organoid.
